(19) Europäisches Patentamt
European Patent Office
Office européen des brevets

(11) **EP 3 580 345 B1**

(12) **EUROPEAN PATENT SPECIFICATION**

(45) Date of publication and mention
of the grant of the patent:
**07.09.2022 Bulletin 2022/36**

(21) Application number: **18704225.4**

(22) Date of filing: **08.02.2018**

(51) International Patent Classification (IPC):
**C12P 3/00** $^{(2006.01)}$

(52) Cooperative Patent Classification (CPC):
**C12P 3/00**

(86) International application number:
**PCT/EP2018/053115**

(87) International publication number:
**WO 2018/146167 (16.08.2018 Gazette 2018/33)**

(54) **PHOTOAUTOTROPHIC AND SUSTAINABLE PRODUCTION OF HYDROGEN IN ALGAE**

PHOTOAUTOTROPHE UND NACHHALTIGE HERSTELLUNG VON WASSERSTOFF IN ALGEN

PRODUCTION PHOTOAUTOTROPHE ET DURABLE D'HYDROGÈNE DANS DES ALGUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2017 EP 17155168**

(43) Date of publication of application:
**18.12.2019 Bulletin 2019/51**

(73) Proprietor: **Biological Research Centre
6726 Szeged (HU)**

(72) Inventors:
• **NAGY, Valéria
6726 Szeged (HU)**
• **TÓTH, Szilvia Zita
6726 Szeged (HU)**

(74) Representative: **Gassner, Birgitta et al
REDL Life Science Patent Attorneys
Donau-City-Straße 11
1220 Wien (AT)**

(56) References cited:
• **DUBINI ALEXANDRA ET AL: "Engineering photosynthetic organisms for the production of biohydrogen", PHOTOSYNTHESIS RESEARCH, SPRINGER NETHERLANDS, DORDRECHT, NL, vol. 123, no. 3, 27 March 2014 (2014-03-27), pages 241-253, XP035452197, ISSN: 0166-8595, DOI: 10.1007/S11120-014-9991-X [retrieved on 2014-03-27]**

• **DEGRENNE B ET AL: "Effect of prolonged hypoxia in autotrophic conditions in the hydrogen production by the green microalgain photobioreactor", BIORESOURCE TECHNOLOGY, ELSEVIER BV, GB, vol. 102, no. 2, 2 August 2010 (2010-08-02), pages 1035-1043, XP028371517, ISSN: 0960-8524, DOI: 10.1016/J.BIORTECH.2010.08.009 [retrieved on 2010-08-06] cited in the application**

• **ROEHL M. CINCO ET AL: "The role of carbon dioxide in light-activated hydrogen production by Chlamydomonas reinhardtii", PHOTOSYNTHESIS RESEARCH., vol. 38, no. 1, October 1993 (1993-10), pages 27-33, XP055393690, DORDRECHT, NL ISSN: 0166-8595, DOI: 10.1007/BF00015058**

• **GERGELY LAKATOS ET AL: "Bacterial symbionts enhance photo-fermentative hydrogen evolution of Chlamydomonas algae", GREEN CHEMISTRY, vol. 16, no. 11, 2014, pages 4716-4727, XP055393150, GB ISSN: 1463-9262, DOI: 10.1039/C4GC00745J**

Remarks:
The file contains technical information submitted after the application was filed and not included in this specification

**Description**

**Field of the Invention**

The present invention relates generally to hydrogen production in algae.

**Background Art**

Hydrogen is a highly efficient and clean energy source. The [Fe-Fe]-type hydrogenases of green algae, located at the acceptor side of photosystem I (PSI), are probably the most active catalysts for hydrogen ($H_2$) production, which, however, are highly sensitive to the $O_2$ produced during photosynthesis. Resolving this antagonism is a major hurdle for the implementation of a sustainable $H_2$ economy.

Among the potential renewable energy sources, photobiological hydrogen production stands out as an appealing choice, because it is carried out by microorganisms in an aqueous environment, possibly without arable land requirement. Algal $H_2$ production may become a genuinely $CO_2$-neutral energy source because upon the combustion of $H_2$, only pure water is produced.

Owe to its very high theoretical efficiency, photobiological $H_2$ production by green algae has been studied for decades. A major hurdle in reaching a commercially viable $H_2$ production in green algae is the $O_2$ sensitivity of the hydrogenase. Engineered $O_2$-insensitive hydrogenases would, however, lead to less efficient photosynthesis under normal growth conditions, because the reducing power generated by the photosynthetic electron transport chain would be partially used for $H_2$ production instead of producing photosynthates. Alternatively, anaerobiosis can be established by downregulating photosystem II (PSII) activity, most commonly by sulphur deprivation in *Chlamydomonas reinhardtii* (Antal et al., 2015). However, sulphur deprivation is unspecific and results in the degradation of photosynthetic complexes and cell death on the timescale of days (Zhang et al., 2002). $H_2$ production induced by sulphur deprivation is also strongly dependent on acetate as an organic carbon source, meaning that *in sensu stricto* it is not a photoautotrophic process (Fouchard et al. 2005; Kosorouv et al., 2003).

$H_2$ production can be induced also by anaerobiosis in nutrient-replete Tris-acetate-phosphate (TAP) medium, which consists of incubating Chlamydomonas cultures in the dark for a few hours in an $O_2$-free atmosphere and exposing them to relatively low light. Under these conditions, $H_2$ production is temporary, because the (evolved $O_2$ inhibits the hydrogenases on the timescale of minutes; Hemschemeier et al., 2009). By incubating the cultures under very low light conditions, a balanced $O_2$ evolution and respiration can be established, resulting in low-yield $H_2$ production lasting for several days (Scoma et al., 2014). This type of $H_2$ production depends largely on photosynthesis and partly on fermentative processes (Scoma et al., 2014). The yield of $H_2$ production can be increased by enhancing acetate respiration (Jurado-Oller et al., 2015), which, however, represents a severe loss in efficiency, since for each $H_2$ produced, an equivalent amount of substrate is respired (Hallenbeck and Benemann 2002). Therefore, a direct and efficient conversion of solar energy to $H_2$ in a carbon-neutral way, i.e. direct biophotolysis is required for commercial viability (Dubini and Ghirardi 2015, Scoma and Tóth 2017). Anaerobiosis-induced $H_2$ production has been observed in photoautotrophic (i.e. acetate-free, $NaHCO_3$-supplemented) cultures as well, in which $H_2$ production lasted for several days at low light (Degrenne et al., 2011), although with a very low efficiency as compared to the widely used sulphur-deprivation method in TAP medium.

Cinco et al. (1993) demonstrated that the Calvin-Benson cycle competes with $H_2$ production. Anaerobiosis was maintained by intensive flushing with ultrapure helium and dark-light cycles of 1.5-1 h were used to maintain anaerobiosis. Lakatos et al. (2014) disclose use of bacterial symbionts to decrease the amount of $O_2$ in mixed algal-bacterial cultures of *Chlamydomonas* sp. MACC-549 and *Chlamydomonas reinhardtii* CC-124. In the mixed algal-bacterial cultures, bacteria are used for consuming the produced $O_2$. Therefore, organic carbon source is required to support bacterial growth and metabolism.

Thus, there is still the need for an improved method for producing $H_2$ in algae.

**Summary of invention**

It is the objective of the invention to provide a method for improving photobiological hydrogen production in algae.

The objective is solved by the subject matter as claimed and further described herein.

The present approach stems from the ultimate physiological role of the highly efficient hydrogenases found in green algae. Their primary function is to serve as a safety valve upon the induction of photosynthesis in hypoxia (Godaux et al. 2016); once the photosynthetic apparatus is fully functional, the hydrogenases become inactive by the evolved $O_2$. By preventing the activation of the Calvin-Benson-Bassham (CBB) cycle upon a dark-to-light transition (by omitting organic or inorganic carbon sources from the medium), the photosynthetic electron transport chain remains largely reduced, resulting in low $O_2$ evolution and sustained hydrogenase activity

Therefore, the present invention relates to a method of hydrogen gas generation, comprising the steps of:

i. culturing under anaerobic conditions in the dark a green alga species selected from the group consisting of *Chlamydomonas sp., Chlorella sp.,* or *Scenedesmus sp.* which are capable of expressing one or more types of hydrogenase enzymes (induction phase),

ii. subsequently culturing the green alga under illuminated conditions in a medium substantially devoid of a carbon sources (production phase), whereupon activation of the CBB cycle in the algae is prevented, wherein substantially devoid of organic or inorganic carbon sources refers to a $CO_2$ content of less than 0.03% in the headspace of the culture, and wherein the light intensity is between 15 - 3100 $\mu$mol photons m$^{-2}$s$^{-1}$;

iii. removing the produced oxygen, and

iv. collecting the evolved hydrogen gas.

One embodiment of the invention relates to the method as described herein wherein steps ii, iii, and iv are carried out simultaneously and continuously.

A further embodiment of the invention relates to the method as described herein, wherein the alga is capable of expressing hydrogenase enzymes in the chloroplast.

A further embodiment of the invention relates to the method as described herein, wherein the hydrogenase is a [Fe-Fe]-type hydrogenase.

A further embodiment of the present invention relates to a method of hydrogen gas generation, comprising the steps of:

i. culturing green alga species selected from the group consisting of *Chlamydomonas sp., Chlorella sp.,* or *Scenedesmus sp.* capable of expressing one or more types of hydrogenases in the chloroplast in a liquid medium containing organic carbon sources (acetate, for instance) or supplemented with inorganic carbon sources ($NaHCO_3$ or $CO_2$, for instance), and

ii. incubating the green alga culture under anaerobic conditions in the dark (induction phase), whereupon activation of the CBB cycle in the algae is prevented, wherein substantially devoid of organic or inorganic carbon sources refers to a $CO_2$ content of less than 0.03% in the headspace of the culture, and wherein the light intensity is between 15 - 3100 $\mu$mol photons m$^{-2}$s$^{-1}$;

iii. subsequently exposing the alga culture to illumination in a medium substantially devoid of organic and inorganic carbon sources (production phase),

iv. removing the produced oxygen, and

v. collecting the evolved hydrogen gas.

A further embodiment of the invention relates to the method as described herein, wherein the alga is *Chlamydomonas sp., Chlorella sp., Auxenochlorella sp., Tetraselmis (Platymonas) sp.,* or *Scenedesmus sp.*

In one embodiment of the invention, the alga is *Chlamydomonas reinhardtii, Chlorella sorokiniana,* or *Chlorella vulgaris.*

In a further embodiment of the invention, the *Chlamydomonas reinhardtii* strain is CC-124, CC-409, or tla3.

A further embodiment of the invention relates to the method as described herein, wherein oxygen is removed mechanically, chemically or biologically.

A further embodiment of the invention relates to the method as described herein, wherein oxygen absorbent is added to the gas phase or to the culture medium.

A further embodiment of the invention relates to the method as described herein, wherein oxygen is taken up by non-photosynthetic organisms.

A further embodiment of the invention relates to the method as described herein, wherein the oxygen absorbent is iron-based oxygen absorbent, a cobalt complex, or a copper-, palladium- or alumina containing catalyst.

A further embodiment of the invention relates to the method as described herein, wherein the emitted $CO_2$ is removed.

A further embodiment of the invention relates to the method as described herein, wherein activation of the CBB cycle in the alga is prevented by lack of organic or inorganic carbon sources in the medium.

A further embodiment of the invention relates to the method as described herein, wherein the medium of step ii) is limited in any organic or inorganic carbon source during the $H_2$ production phase.

In one embodiment of the invention the medium of step ii) does not contain $CO_2$, carbonate and/or acetate. Specifically, the medium is devoid of $CO_2$, carbonate and/or acetate.

A further embodiment of the invention relates to the method as described herein, wherein the medium of step ii) is a tris-phosphate (TP) or a high-salt (HS) medium.

A further embodiment of the invention relates to the method as described herein, wherein the headspace of the culture contains less than 300 ppm $CO_2$, or less than 30 ppm $CO_2$, or less than 3 ppm $CO_2$.

A further embodiment of the invention relates to the method as described herein, wherein the culture is illuminated by solar radiation or by an artificial light source.

A further embodiment of the invention relates to the method as described herein, wherein the culture exposed to light intensities of about 30 - 500 $\mu$mole photons m$^{-2}$s$^{-1}$ , 50 - 400 $\mu$mole photons m$^{-2}$s$^{-1}$ , 75 - 350 $\mu$mole photons m$^{-2}$s$^{-1}$.

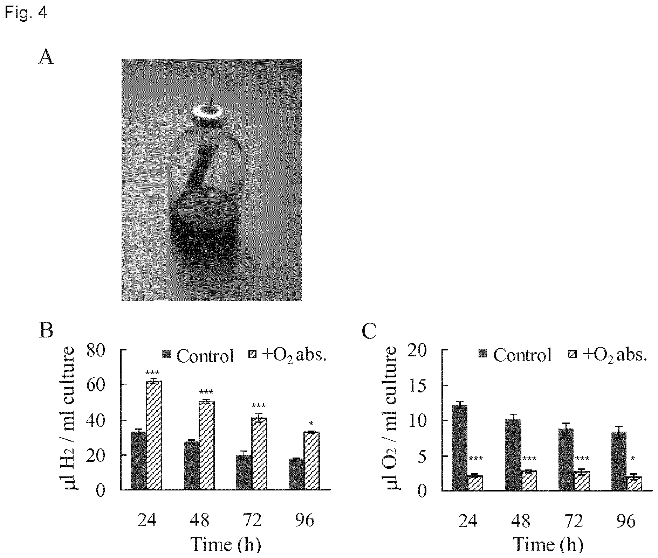

A further embodiment of the invention relates to the method as described herein, wherein thereby at least 0.5 mL/h L hydrogen are produced. According to the invention at least 100 mL hydrogen per L culture are produced in 96 hours starting from the beginning of anaerobic induction (see Fig. 4).

A further embodiment of the invention relates to the method as described herein, wherein hydrogenase enzyme expression in the alga is increased in the induction phase due to anoxia.

A further embodiment of the invention relates to the method as described herein, wherein $O_2$ and/or $CO_2$ are removed in the induction and/or production phase.

A further embodiment of the invention relates to the method as described herein, wherein the alga culture is regenerated after the hydrogen production phase, by providing conditions enabling photosynthesis, $CO_2$ fixation and growth to occur.

## Brief description of drawings

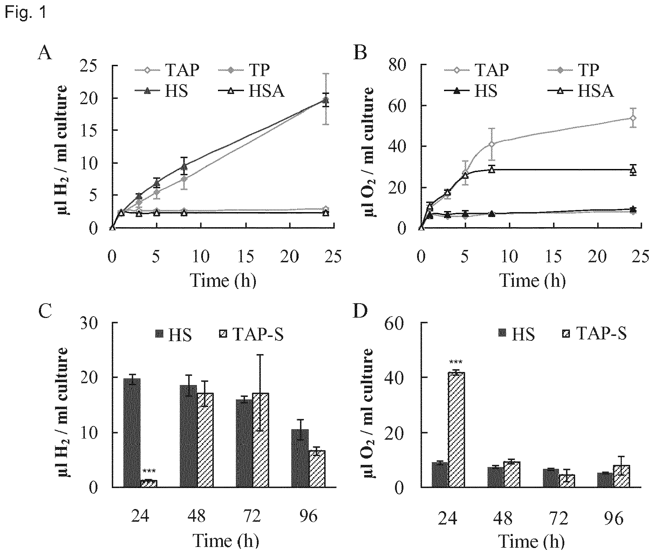

Figure 1: $H_2$ and net $O_2$ productions in Chlamydomonas cultures (50 $\mu$g chl(a+b)/ml, at c. 320 $\mu$mole photons m$^{-2}$s$^{-1}$) as determined in the headspaces of sealed cultures using gas chromatography. $H_2$ (a) and net $O_2$ (b) productions in CC-124 Chlamydomonas cultures in acetate-containing (TAP, HSA) and acetate-free media (TP, HS) following 4-hour incubation under anaerobic conditions in the dark (4 hours darkness with 3 x 10 min $N_2$ flushing). Daily $H_2$ (c) and daily net $O_2$ (d) productions in Chlamydomonas cultures in sulphur-free acetate-containing (TAPS) and sulphur-containing minimal media (HS). The alga cultures were flushed every 24 hours with $N_2$ after determining the gas concentrations in the headspaces of the sealed bottles in order to avoid excessive $H_2$ accumulation in the headspace. Mean values ($\pm$SEM) are each based on 6 biological replicates. Statistical significance levels are presented relative to the Chlamydomonas culture subjected to dark anaerobic incubation in HS medium as *** p<0.001.

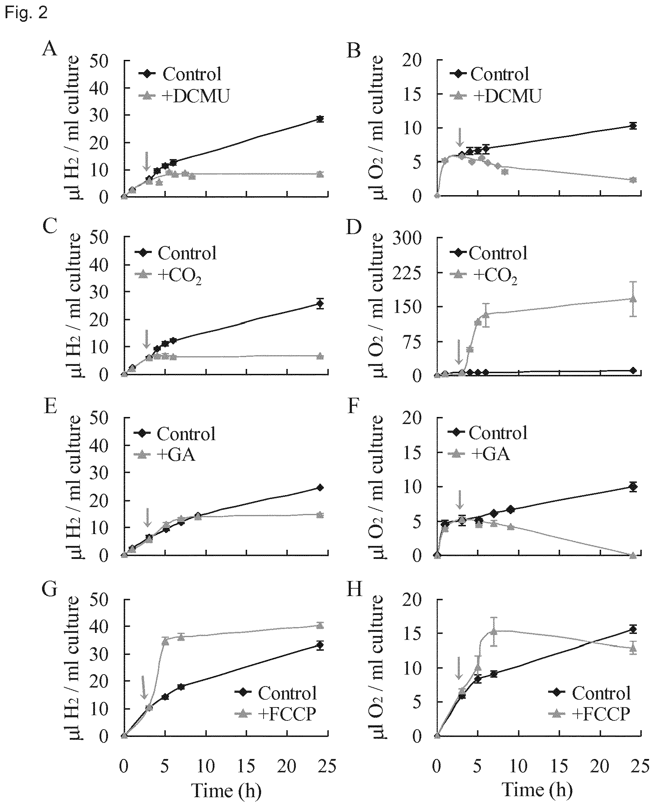

Figure 2: $H_2$ (a, c, e, g) and net $O_2$ (b, d, f, h) productions in anaerobically-treated Chlamydomonas cultures in HS medium, at 320 $\mu$mole photons m$^{-2}$s$^{-1}$, upon the addition of the PSII inhibitor DCMU (a,b), 2% $CO_2$ (c, d), the CBB cycle inhibitor glycolaldehyde (GA) (e, f), and the ionophore carbonilcyanide p-triflouromethoxyphenylhydrazone (FCCP) (g, h). The time point at which the various treatments were applied is indicated by an arrow. Mean values ($\pm$SEM) are each based on 4 or 5 biological replicates.

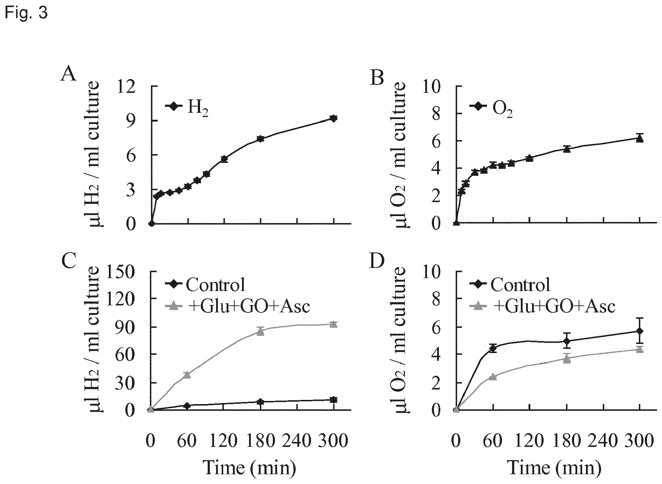

Figure 3: *In vivo* $H_2$ (a,c) and net $O_2$ productions (b,d) in anaerobically-treated Chlamydomonas cultures in HS medium at 320 $\mu$mole photons m$^{-2}$s$^{-1}$ as determined in the headspaces of sealed cultures using gas chromatography. (a, b), detailed time courses (c,d), the effects of glucose (Glu, 2 mM), glucose oxidase (GO 0.2 mg/ml) and ascorbate (Asc, 1 mM) added following the dark anaerobic incubation. Mean values ($\pm$SEM) are each based on 4 to 8 biological replicates.

Figure 4: The effect of iron-salt-based $O_2$ absorbent placed in the headspace (a) on the daily $H_2$ (b) and daily net $O_2$ (c) productions in anaerobically-treated Chlamydomonas cultures (4 hours darkness with 3x10 min $N_2$ flushing) in HS medium at 320 $\mu$mole photons m$^{-2}$s$^{-1}$. The cultures were flushed with $N_2$ for 10 min every 24 hours after determining the gas concentrations in the headspaces of the sealed bottles. Mean values ($\pm$SEM) are each based on 11-15 biological replicates. Statistical significance levels are presented relative to the Chlamydomonas culture subjected to dark anaerobic incubation in HS medium as * p<0.05; *** p<0.001.

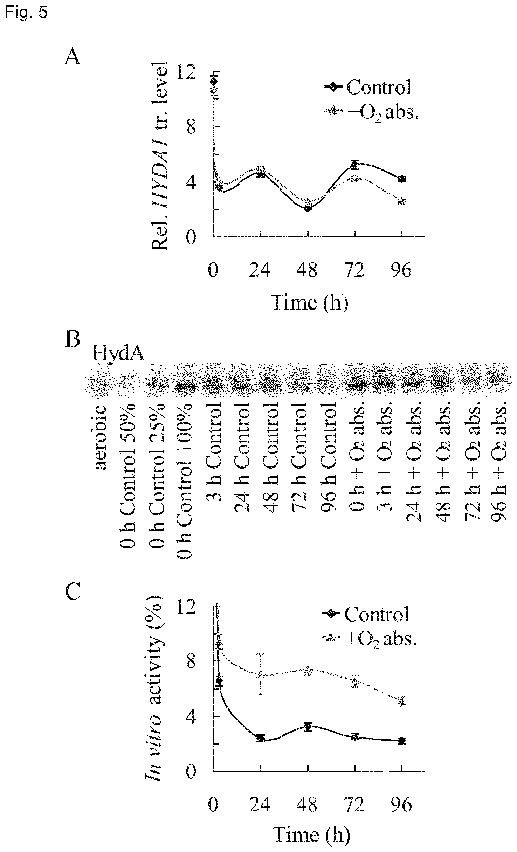

Figure 5: Relative *HYDA1* expression and HydA activity in the absence and presence of an iron-salt-based $O_2$ absorbent in anaerobically-treated Chlamydomonas cultures, as determined by quantitative RT-PCR (a), western blot analysis (b), and *in vitro* hydrogenase activity (c) during 96 hours of $H_2$ production in HS medium at 320 $\mu$mole photons m$^{-2}$s$^{-1}$. The *HYDA1* expression level is expressed relative to the aerobically cultured controls (a), which was also used as a 0 hour control for the western blot analysis (b). The *in vitro* hydrogenase activity (c) is expressed as a percentage of the activity of cells treated anaerobically for 3 hours in the dark, which had an *in vitro* hydrogenase activity of approximately 500 $\mu$mole $H_2$/mg chl(a+b) h$^{-1}$. Mean values ($\pm$SEM) are each based on 3 or 4 biological replicates.

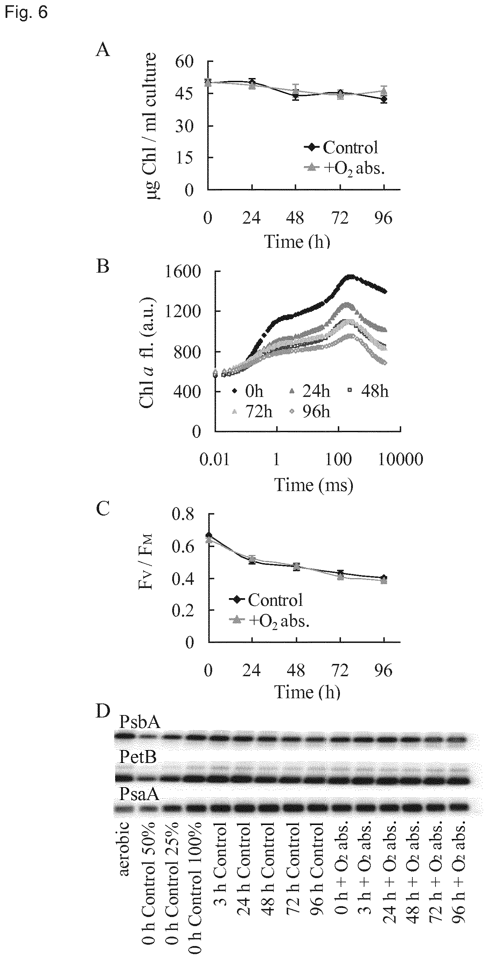

Figure 6: Characterization of the photosynthetic apparatus during 96 hours of $H_2$ production in HS medium at 320 $\mu$mole photons m$^{-2}$s$^{-1}$ in anaerobically-treated Chlamydomonas cultures in the absence and presence of iron-salt-based $O_2$ absorbent, as determined by chl(a+b) content measurements (a), fast chl a fluorescence (OJIP) transients (b), the $F_V/F_M$ fluorescence parameter (c), and western blot analysis for the semi-quantitative determination of PsbA, PetB and PsaA contents, loaded based on equal chl(a+b) contents (d). Mean values ($\pm$SEM) are each based on 3 to 6 biological replicates.

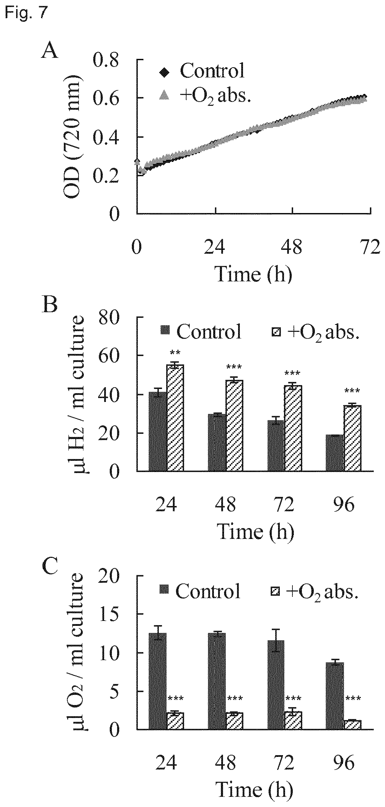

Figure 7: Culture growth during 72 hours following a 96 hour-period of $H_2$ production in HS medium, as determined by measuring optical density at 720 nm (a), and subsequent $H_2$ (b) and net $O_2$ productions (c) in cultures subjected to a second phase of dark anaerobic incubation in the presence and absence of an iron-salt-based $O_2$ absorbent. The cultures were flushed every 24 hours with $N_2$ after determining the gas concentrations in the headspaces of the sealed bottles. Mean values ($\pm$SEM) are each based on 4 biological replicates. Statistical significance levels are presented relative to the Chlamydomonas culture subjected to dark anaerobic incubation in HS medium without $O_2$ absorbent as *** p<0.001.

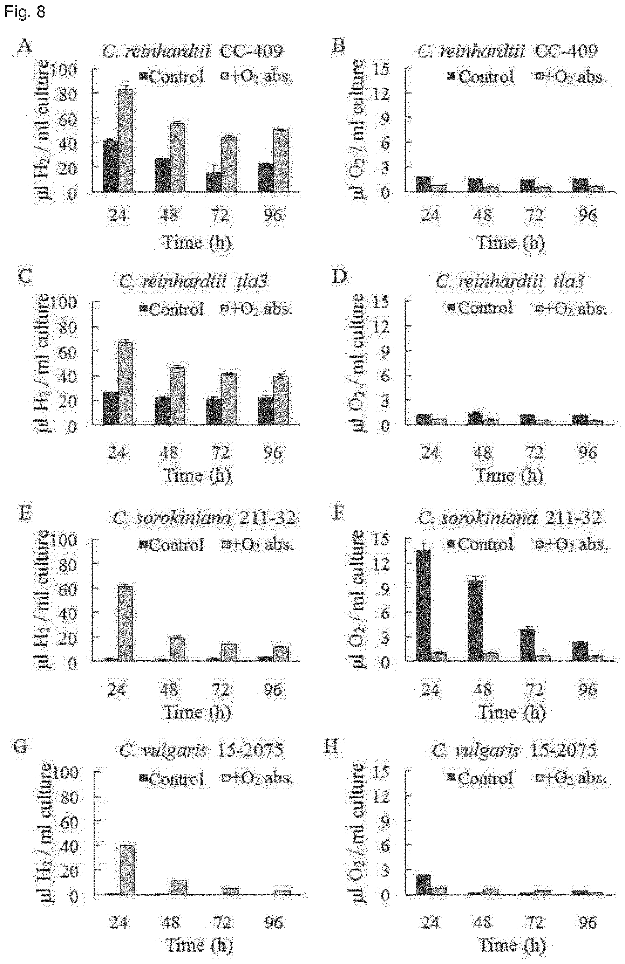

Figure 8: Daily $H_2$ (A, C, E, G) and daily net $O_2$ (B, D, F, H) productions in anaerobically-treated *Chlamydomonas reinhardtii* CC-409 strain (A, B), *Chlamydomonas reinhardtii tla3* mutant (C, D), *Chlorella sorokiniana* 211-32 (E, F), and *Chlorella vulgaris* 15-2075 cultures, in the presence or absence of an iron-salt-based $O_2$ absorbent in the headspaces of the cultures. The culture conditions and treatments are identical as in Fig. 4.

## Description of embodiments

The methods provided herein use algal cells to efficiently generate hydrogen.

Photosynthesis may simply be defined as the conversion and storage of light energy into chemical energy by living organisms. The rate of photosynthesis is affected by environmental factors, including the availability of carbon dioxide ($CO_2$), light intensity, and temperature.

Photosynthesis occurs in two stages. During the light-dependent photosynthetic reactions (also called the light reactions) the energy of light is captured and it is used to generate high-energy molecules, namely NADPH and ATP. During the second phase, called the CBB cycle (formerly known as dark reactions), the high-energy molecules are used up to assimilate $CO_2$ and carbohydrates are synthesized.

Photosynthesis is the entry for nearly all high energy electrons into biogeochemical cycles. Photosynthesis is an electron transport pathway in which five key events occur:

i. light is absorbed by the chlorophyll (chl)-containing antenna complexes of the photosynthetic apparatus, followed by the transfer of excitation energy to the reaction centers chl of PSII and PSI (P680 and P700, respectively);

ii. P680 and P700 are moved to a high energy state and become oxidized by transferring electrons to their respective acceptor molecules;

iii. The oxidizing power in PSII is used by the oxygen evolving complex (OEC) to split water, which is the ultimate source of electrons and protons for the photosynthetic electron transport;

iv. The electrons originating from water are transferred to $P680^+$ in PSII, moved to the plastoquinone pool, the cytochrome $b_6f$ complex and to PSI;

v. the electrons are transferred to ferredoxin and the enzyme ferredoxin-NADP($^+$) oxidoreductase generates NADPH to be used in the CBB cycle.

vi. During the electron transport processes, a proton gradient is generated between the stromal and the luminal sides of the thylakoid membrane, which is used to generate ATP.

The methods provided herein were designed using the principle that the electrons originating from water splitting can be used in chemical redox reactions, such as reducing two protons to molecular hydrogen in green algae by hydrogenases expressed in the chloroplast.

The methods provided herein use hydrogenases to accomplish this. Hydrogenases are enzymes which catalyze the reaction

$$2H^+ + 2e^- \rightleftarrows H_2.$$

This reaction is reversible, and there are hydrogenases in nature that catalyze both the forward and reverse reaction. Hydrogenase enzymes found in green algae are oxygen sensitive and get irreversibly inhibited as $O_2$ accumulates originating from photosynthesis. Hydrogen production acts as an electron "safety valve" to avoid the over accumulation of electrons at the acceptor side of PSI, thus to avoid photoinhibitory damage during the induction of photosynthesis upon a dark-to-light transition. Thus, in algae, photosynthesis can be linked to $H_2$ production.

[Fe-Fe]-type hydrogenases found in green algae are one of the most active molecular catalysts known for $H_2$ production. The green alga *C. reinhardtii* has two [Fe-Fe]-type hydrogenase paralogues (HYDA, hydrogenase enzyme), called HydA1 and HydA2, the turnover rate of the major form HydA1 is several thousand per second, about 100-fold higher than that of other types of hydrogenases (Rousset and Liebgott 2014).

Hydrogenases of green algae are located in the chloroplast stroma and become highly expressed under anaerobic conditions established for instance in the dark; upon illumination, electrons from PSI are directed to the hydrogenases, thereby preventing the over-reduction of the photosynthetic electron transport chain. By this reaction, hydrogenases also promote the light-induced increase of stromal pH necessary for the activation of the CBB cycle and it ultimately supports ATP formation. Thus, hydrogenases play an essential role upon the induction of photosynthesis in green algae (Godaux et al., 2016). Once the electron transport is fully functional, the $O_2$ evolved by PSII reacts with HydA1, leaving an inactive [4Fe-4S] subcluster state (Swanson et al., 2015); $O_2$ also inhibits hydrogenase expression (Eivazova and Markov 2012), possibly in order to avoid wasting the absorbed light energy once the CBB cycle is fully functional.

The CBB cycle is inactive in the dark and is activated upon illumination. The CBB cycle remains inactive upon illumination if the medium is substantially devoid of a carbon source, e.g. by omitting $CO_2$ or acetate from the medium. Therefore, the $H_2$ production medium does not contain acetate, $CO_2$, or carbonate (e.g. $NaHCO_3$). Surprisingly, it was found by the inventors that keeping the CBB cycle inactive by substrate limitation is sufficient to maintain low $O_2$ levels in the culture medium, enabling partial preservation of hydrogenase activity and allowing sustained $H_2$ production.

As used herein, the terms hydrogen gas ($H_2$) and hydrogen are used interchangeably. As used herein, the terms oxygen gas ($O_2$) and oxygen are used interchangeably. The term carbon dioxide and $CO_2$ are used interchangeably.

As used herein, the word "culture" refers to the maintenance of living cells in media that is conducive to their ongoing viability. Many media are conducive to not only viability, but also growth under the appropriate environmental conditions. As used herein, the term "growth" is defined as expansion of the culture, i.e. increase of number of organisms in the culture, over a defined period of time. The most common growth media include inorganic salts to which some vitamins and acetate or $NaHCO_3$ may be added and which will include sulfur as a component. The culture may be liquid or solid; in the latter case, agar is used to solidify the media. Culturing may be done on a laboratory scale in various vessels or on a commercial scale using photobioreactors.

As used herein, the term "medium" refers to a medium for maintaining the algae in a viable state, with or without growth, for the duration of the culture period. In one embodiment, the culturing medium is substantially devoid of carbon, so that culture of the photosynthetic algae in the reduced-carbon culture medium results in substrate-limited CBB cycle, inferring over reduction of the photosynthetic electron transport chain, thus a limited oxygen-evolving activity, leading to hypoxic or, ostensibly anaerobic conditions.

As used herein, the term "anaerobic" or "anaerobic conditions" refer to an environment with little or no available oxygen and is used interchangeably with the term hypoxic.

There are several media recipes available for culturing green algae. Media for culturing alga are for example TAP-medium (Tris-Acetate-Phosphate), HS-medium (*Sueoka's high salt medium*), f/2 medium, BG-11 medium, Bold's Basal Medium (BBM), ATCC medium, WC Medium, Alga-Gro Lake-Water medium, Polytoma medium, M-8 medium, Kolkwitz medium, LC Oligo medium, TM medium, OM medium, SK medium, Asp medium. Suitable media for producing hydrogen gas by the alga according to the present invention have to be substantially devoid of a carbon source. Specifically, the medium is devoid of acetate, $CO_2$, or carbonate.

As used herein, "substantially devoid of carbon source" means that the headspace of the culture generally contains less than 300 ppm (0.03%) $CO_2$, preferably less than 30 ppm $CO_2$, and most preferably less than 3 ppm $CO_2$. In case of organic carbon sources, e.g. acetate, its concentration in the culture medium is less than 1%, preferably less than 0.1% and most preferably 0.01%. In the case of bicarbonate ($NaHCO_3$) or other inorganic carbon sources, its concentration in the culture medium is less than 20 mM, preferably less than 2 mM and most preferably less than 0.2 mM.

The additional application of an iron-based $O_2$ absorbent resulted in yields that are four-fold greater as observed for the standard sulphur deprivation procedure in our experimental setup.

As described herein, the alga has at least one type of hydrogenase. Algae suitable for use include naturally occurring algae (wild type), cultivated strains of algae, strains of algae resulting from hybridization and selection processes, naturally occurring or artificially induced mutagenesis, genetically modified algae including CRISPR-related technologies, such as for example CRISPR/Cas9 or CRISPR/cpf1 technology, and transient induction or repression of specific sets of genes, having specifically enhanced traits. Algae having specific characteristics may also be used, for example, mutant algae having modified photosensitivity or modified components of the photosynthetic apparatus and hydrogenases. Mutant algae and methods for their production and screening are disclosed in, *inter alia,* US20100273149 and US20090221052.

Several microalgal species, including *Chlamydomonas reinhardtii,* are known for their ability to photo-evolve $H_2$ in anaerobiosis as the result of electron transport from ferredoxin to chloroplast-located hydrogenases, which catalyze the reversible reduction of protons into molecular hydrogen. Due to the extreme $O_2$ sensitivity of the hydrogenase, $H_2$ evolution in the light usually is a transient phenomenon, lasting for minutes, that occurs after pre-incubation of the algae in anoxia.

The induction of a chloroplast Fe-Fe hydrogenase, mediating the light-driven reduction of $H^+$ to $H_2$ in the chloroplast stroma, is one of the most enigmatic characteristics of the anaerobic response of green algae, specifically of C. *reinhardtii.*

Thus, a further example as described herein relates to the method, wherein the alga is capable of expressing at least one type of hydrogenase enzyme in the chloroplast. According to our present knowledge, [Fe-Fe]-type hydrogenases can catalyze this reaction in the chloroplast.

In general, [Fe-Fe]-hydrogenase enzymes characteristically possess a catalytic site consisting of a bimetallic center containing two Fe and two S atoms (2Fe2S center). The chemistry of the [Fe-Fe]-hydrogenase catalytic core is reactive with respect to hydrogen, typically possessing very high hydrogen-production and/or oxidation rates. However, this same catalytic core is also highly sensitive to inactivation by oxygen.

As used herein, the terms alga, algae or the like, refer to a polyphyletic group of photosynthetic organisms, excluding land plants. As used herein, following the classification of Leliaert et al. (2011), green algae may belong to the Chlorophyta or the Streptophyta phylum of Viridiplantae, excluding land plants. Green algae are without roots, stems or leaves, they may be unicellular or multicellular; they contain chl and carry out photosynthesis.

In some examples, green algae, belonging to Eukaryota-Viridiplantae-Chlorophyta- *Chlorophyceae* and *Trebuxiophyceae* classes are used. Non-limiting examples include the genuses *Chlamydomonas, Tetraselmis (Platymonas), Scenedesmus, Chlorella* and *Auxenochlorella.*

In some specific examples, the alga is selected from the group consisting of *Platymonas subcordiformis, Chlamydomonas caudate Wille, Chlamydomonas elegans G.S. West 1915, Chlamydomonas moewusii, Chlamydomonas nivalis, Chlamydomonas ovoidae, Chlamydomonas reinhardtii, Chlorella autotrophica, Chlorella coloniales, Chlorella lewinii, Chlorella minutissima, Chlorella pituita, Chlorella pulchelloides, Chlorella pyrenoidosa, hlorella rotunda, Chlorella singularis, Chlorella sorokiniana, Chlorella variabilis, Chlorella volutis, Chlorella vulgaris, Auxenochlorella protothecoides,* or *Auxenochlorella sp. P-1015.*

In some specific embodiments, *C. reinhardtii,* belonging to Chlamydomonales, is used. In other specific embodiments, the strain *Chlamydomonas reinhardtii* CC-124*,* CC-409 or tl3 is used.

General methods for culturing of Chlamydomonas are well known in the art, and are described in detail in The Chlamydomonas Sourcebook (Harris, 2009), the contents of which are incorporated herewith by reference). General methods for photoproduction of hydrogen in algae are described in detail in Hemschemeier et al. (2009), the contents of which are incorporated herewith by reference.

Due to the $O_2$ sensitivity of the hydrogenases, it is preferred that the evolved $O_2$ is removed. The removal could be biological, mechanical or chemical by adding oxygen absorbents. Cellular respiration may also contribute to the decrease in the $O_2$ level in the culture medium. Suitable chemical absorbents are a finely divided metal powder such as iron that is coated with a halogen salt such as sodium chloride, or iron powder optionally in combination with salt and/or electrolyte. Alternatively, cobalt complexes (Sundberg et al., 2014), copper-, palladium- or alumina containing catalysts for oxygen removal may also be used.

The chemical absorbent may be added to the gas phase or possibly to the culture medium. A proper mixing of the culture is necessary for efficient oxygen removal.

Alternatively, the alga may be co-cultured with non-photosynthetic organism which is capable of taking up $O_2$. Examples for such non-photosynthetic microorganism are *Escherichia coli, Ralstonia eutropha, Rhodococcus sp., Brevundimonas sp., Leifsonia sp* (Lakatos et al., 2014). When using co-cultures, the evolved $CO_2$ may need to be removed in order to prevent the activation of the CBB cycle in the alga. However, in the mixed algal-bacterial cultures, organic carbon source such as acetate is required to support bacterial metabolism. When the organic carbon source is depleted, the bacterial cultures may decay or collapse and finally the bacteria may die and the culture becomes aerobic again, thus $H_2$ production is ceased.

During the production phase the alga culture is illuminated by solar radiation or by an artificial light source.

Illumination is typically provided externally. Illumination can be solar illumination or artificially produced and provided. For sunlight, the methods of the present invention may be practiced outdoors, indoors (i.e. in greenhouses or similar) or applying the combination of the two, utilizing the sunlight available during the daytime. Full sunlight may provide illumination up to approximately 2200 $\mu$mol photons m$^{-2}$s$^{-1}$. Additional artificial illumination can be added during the dark period or when the natural light is insufficient.

Artificial illumination can be provided by incandescent, fluorescent, LED (light emitting diode) or other sources. In some embodiments, the illumination is via fluorescent or LED lighting, in order to minimize the costs and to reduce the amount of heat generated during intense illumination. During illumination for photoproduction of hydrogen, the light may be from an artificial source or natural sunlight, and must be sufficient for photosynthesis to occur. In one embodiment the light intensity is between 15 - 3100 $\mu$mole photons m$^{-2}$s$^{-1}$ and all ranges within this range such as 20 - 3000, 30 - 2500, 50 - 2000, and so on. In some embodiments the light intensity is between 30 - 500 $\mu$mole photons m$^{-2}$s$^{-1}$, 50 - 400 $\mu$mole photons m$^{-2}$s$^{-1}$, or 75 - 350 $\mu$mole photons m$^{-2}$s$^{-1}$. Illumination is continued for up to 144 hours but may be for a lesser period such as 24, 48, 64 or 96 hours or longer periods, such as 192, 240 and 288 hours. The periods of illumination may also be intermitted with dark periods of various lengths. The dark period may last for a few hours but may also last overnight so that the protocol would fit the natural light-dark cycles. Accordingly, the $H_2$ production phase may take place during the natural light period. However, the natural dark period may be shortened by artificial illumination, which may be continuous or intermittent (short periods of light pulses). The intensity of artificial illumination may be low (a few $\mu$mol photons m$^{-2}$s$^{-1}$) or high (up to 5000 $\mu$mol photons m$^{-2}$s$^{-1}$).

In some embodiments, illumination during the production phase of hydrogen is 320 $\mu$mol photons m$^{-2}$s$^{-1}$ $\pm$20% at the level of the vials containing the alga cultures. Towards the end of the light period, $CO_2$ may be added to stimulate photosynthesis and allow recovery of the alga from $H_2$ production. $CO_2$ addition may last for a few hours or only a few minutes, and the excess $CO_2$ may need to be removed before the dark period starts.

In some embodiments, intensity of illumination is varied during different stages/phases of the method. For example, during the growth phase of the algae in the induction phase illumination it can be in the range of 15 - 100 $\mu$ mole photons m$^{-2}$s$^{-1}$.

In some embodiments of the present invention, the algal culture is kept at ambient temperature. In other embodiments, the temperature is controlled, for example, to maintain about 25° C in the culture. Methods for temperature control in bioreactors are well known in the art.

The method of the invention may be carried out in a flask, a vessel, or a suitable bioreactor which can further comprise means for stirring the algal culture, means for temperature control of the algal culture, means for sampling the culture and the gas from the algal containment or gas collection means, and suitable means for removing oxygen. The culture vessels are preferably fashioned from a transparent or translucent material, to allow penetration of light.

Hydrogen produced and collected by the methods and systems of the present invention can be stored as compressed gas, liquefied gas, by cryopreservation, chemically as compounds that release hydrogen upon heating, and the like. Stored hydrogen can be used for ammonia production, conversion of petroleum to lighter fuels (hydrocracking), in fuel cells, and the like.

The novel process significantly boosts the hydrogen gas production by the algal culture. In some embodiments of the invention at least 0.5 mL/h L hydrogen over a time period of 48, 72, or 96 hours are produced by the method as described herein. In some embodiments least 0.6, 0.7, 0.8, 0.9, 1.0, 1.1, 1.2, 1.3, 1.4, 1.5, 1.6, 1.7, 1.8 or even 1.87 mL/h L hydrogen are produced by the inventive method. The most limiting factor for the method is the removal of the evolving $O_2$. Thus, the production of even 30 mL/h L hydrogen should be feasible.

Hydrogenase expression in algae could be induced by culturing the alga under anaerobic conditions and in the absence of light (induction phase).

In some embodiments of the present invention hydrogenase expression in the alga is increased in the induction phase by culturing the alga under anaerobic conditions in the absence of light for a period of about at least 10 min, 1 hour, 2 hours, 3 hours or 4 hours.

In some embodiments of the present invention oxygen and optionally $CO_2$ are removed during the induction phase.

Algal cultures can be reused following cessation or significant loss of efficiency of hydrogen photo-production. The algae can be regenerated by dilution with fresh medium, preferably without acetate or other organic carbon sources, but by providing $CO_2$ and sufficient illumination for the algae to re-establish vigorous photosynthesis and to accumulate a large biomass, followed by another cycle of culture for hydrogen photo-production according to methods of the present invention. The surplus of produced biomass may be also used for other biotechnological or non-biotechnological purposes.

A further embodiment of the invention relates to the method as described herein, wherein the alga biomass is regenerated after hydrogen production and reused again. In some embodiments the biomass is regenerated at least twice, threefold, or at least fourfold.

Larger cell density would be advantageous to have a larger respiration ratio per culture volume, which may also contribute to $O_2$ consumption. Thus, dense cultures of about 50 $\mu$g chl/mL culture may be preferred. However, the method may work with less dense or even denser cultures as well.

**Material and Methods**

Determination of net $H_2$ and $O_2$ production by gas chromatography

The net amounts of $H_2$ and $O_2$ produced by the cells were determined by taking 250 $\mu$l aliquot from the gas phase of the cultures with a gas tight microsyringe. These samples were injected manually into an Agilent 6890N gas chromatograph (GC) equipped with a HP-PLOT Molesieve 5 Å column (30 m*0.53 mm*0.25 $\mu$m) and a TCD detector. The oven temperature was 30 °C. The carrier gas was argon, and a linear velocity of 115 cm/s was used. The bottle was flushed with $N_2$ gas daily after the determination of the gas production.

*In vitro* hydrogenase activity assay

*In vitro* hydrogenase activity was measured after the dark anaerobiosis treatment and during the course of $H_2$ production in the light, as described in Hemschemeier et al. (2009). The assay was carried out in 13.5 ml serum vials at 37 ° C and the reaction mixture consisted of 1 ml of 100 mM potassium phosphate buffer, pH 6.8, 80 $\mu$l of deionized

water, 200 $\mu$l of 10% Triton X-100, 20 $\mu$l of 1 M methylviologen, 200 $\mu$l of anaerobic 1 M sodium dithionite and 200 $\mu$l of algal culture. The $H_2$ concentration in the head-space was measured by GC every 15 min and fitted with linear regression. Results are the mean value of tests performed in at least four replicates.

RNA isolation and qRT-PCR analysis to assess *HYDA1* transcript level

For RNA isolation, 1 ml culture, containing approximately 50 $\mu$g chl(a+b), was collected and the Direct-Zol RNA kit was used, following the recommendations of the manufacturer (ZymoResearch). To remove contaminating DNA from the samples, the isolated RNA was treated with DNaseI (ZymoResearch). RNA integrity was checked on a 1% (w/v) MOPS gel. Reverse transcription was primed with oligo dT using 1 $\mu$g of total RNA and SuperScript III reverse transcriptase (Life Technologies). To confirm the absence of DNA contaminations, an aliquot of the RNA sample was used without reverse transcriptase.

Real-time qPCR analysis was performed using an Applied Biosystems Prism 7900HT Fast Real Time PCR System using HOT FIREPol® EvaGreen® qPCR Mix Plus (ROX) (Solis BioDyne). Primers for real-time qPCR analysis were designed using the NCBI Primer Blast Tool (http://www.ncbi.nlm.nih.gov/tools/primer-blast/). The melting temperature was set to 60° C, the amplicon length was set between 100 and 130 bp. To ensure correct normalization of the *HYDA1* (Cre03.g199800) transcript level, we tested the expression levels of several potential reference genes. The three reference genes showing the most stable expression during $H_2$ production were *bTUB2* (Cre12.g549550), *ACTIN* (Cre13.g603700) and *UBQ* (XP_001694320). The primers for *HYDA1* were GGCGAGTGGGACAATCCAAT and TGCCCGTGAACAGCTCATAG; for the reference genes, see Vidal-Meireles et al. (2017). The data are presented as fold-change in mRNA transcript abundance of *HYDA1,* normalized to the average of the three reference genes, and relative to the control sample (cultures grown in TAP medium under normal growth conditions). Real-time qPCR analysis was carried out with three technical replicates for each sample and two or three biological replicates were measured; the standard error was calculated based on the range of fold-change by calculating the minimum and the maximum of the fold-change using the standard deviations of the $\Delta \Delta$ Ct.

Fast Chl *a* fluorescence (OJIP) measurements

Fluorescence measurements were carried out at room temperature with a Handy-PEA instrument (Hansatech Instruments Ltd, UK). *C. reinhardtii* cultures were dark-adapted for 15 min and then 3 ml of cell suspension (50 $\mu$g chl(a+b)/ml) was filtered onto a Whatman glass microfibre filter (GF/C) that was placed in a Handy-PEA leaf clip. The alga sample was illuminated with continuous red light (3500 $\mu$mole photons $m^{-2}$ $s^{-1}$, 650 nm peak wavelength; the spectral half-width was 22 nm; the light emitted by the LEDs is cut off at 700 nm by a NIR short-pass filter). The light was provided by an array of three light-emitting diodes focused on a circle of 5 mm diameter of the sample surface. The first reliably measured point of the fluorescence transient is at 20 $\mu$s, which was taken as $F_0$. The length of the measurements was 5 s.

At each time point, 1 ml of culture were collected, spun-down for removal of the supernatant and frozen in liquid nitrogen. The samples were then solubilized with 370 $\mu$l of protein extraction buffer (50 mM Tris/HCl [pH 8.3], 0.25 % Triton X-100, 1 mM dithiothreitol and 1x cOmplete Protease Inhibitor Cocktail [Roche]), sonicated for 30 s, incubated in the dark at 4 °C for 30 min with vigorous shaking, and then centrifuged at 4700 g for 4 min at 4 °C. The supernatant was collected into a new Eppendorf tube and the chl(a+b) content of the protein extract determined. An amount equivalent to 2 $\mu$g chl(a+b) was then mixed with 2x Laemmli buffer and incubated at 75 °C for 10 min. Proteins separated by SDS-PAGE (Perfect Blue Twin Gel System, Peqlab) were transferred to a polyvinylidene difluoride membrane (Hybond P) using a tank blotting system (Cleaver Scientific Ltd). Specific polyclonal antibodies (produced in rabbits) against HydA, PetB, PsbA and PsaA were purchased from Agrisera AB. As secondary antibody, a goat anti-rabbit IgG horseradish peroxidase conjugate was used (Bio-Rad). Immunochemical detection was carried out with the ECL Prime System (GE Healthcare), according to the instructions of the manufacturer.

Efficiency of light to $H_2$ energy conversion ($\eta_c$) was calculated as the ratio of the rate of chemical energy production to the incident or absorbed light power. The chemical energy stored in produced $H_2$ is expressed as the product of the rate of $H_2$ evolution ($k_H$) measured in mole/s units and the higher heating value of $H_2$ ($HHV_H$ = 286000 J/mol). The incident light power was calculated from the total incident light intensity ($E_s$) and the illuminated area (A).

$$\eta_c = k_H \, HHV_H / E_s A$$

When energy conversion efficiency was related to absorbed light energy, the absorbed light power was estimated using the difference of light intensities measured at the bottom surface of serum bottles containing either alga cultures or the same volume of HS medium. The irradiated area was 0.002 $m^2$.

The presented data are based on at least three independent experiments. When applicable, averages and standard errors (SEM) were calculated. Statistical significance was analysed using Student's t-test and the significance level are presented as: * $p < 0.05$; ** $p < 0.01$; *** $p < 0.001$.

**Examples**

The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

**Example 1 - Anaerobically-induced Chlamydomonas cultures in acetate-free media**

CC-124 strain have been chosen for the studies, because it is a relatively efficient $H_2$ producer and has been successfully used under various conditions (e.g. Kosourov and Seibert, 2009; Lakatos et al., 2014, Oncel and Kose 2014; Steinbeck et al., 2015). After growing the cultures for 72 hours in TAP medium, the cells were transferred to culture media with (TAP, HAS) or without acetate (TP, HS) with a chl(a+b) content set at 50 $\mu g/ml$. Hydrogenase expression was induced by anaerobic incubation in the dark during which both $O_2$ and $CO_2$ were removed by $N_2$ flushing. The high chl(a+b) content was set in order to facilitate the establishment of anaerobiosis, PQ-pool reduction and high hydrogenase expression (Chochois et al., 2009).

As opposed to most earlier studies in nutrient-replete conditions, the cultures were subjected to relatively high light intensities (320 $\mu$mole photons $m^{-2}s^{-1}$) after the anaerobic induction. Upon light exposure, substantial $H_2$ production was observed (about 2 $\mu l$ $H_2/ml$ culture) during the first hour in all growth media. Extended illumination did not result in further $H_2$ production in acetate-containing media (TAP and HSA) contrary to acetate-free media (HS, TP), where a sustained $H_2$ production was observed (about 20 $\mu l$ $H_2/ml$ culture in 24 hours, Fig. 1A). The amount of $O_2$ in the headspace of the vials rapidly accumulated in the presence of acetate (Fig. 1B) whereas in its absence, the $O_2$ concentration in the gas phase remained at a low level (about 9 $\mu l$ $O_2/ml$ culture, corresponding to about 0.3% $O_2$ in the headspace). As cultures kept in HSA or TAP had low $H_2$ yields and both TP and HS cultures produced large amounts of $H_2$, the increase in $H_2$ production efficiency could be unambiguously attributed to the absence of acetate. It is also to be noted that the $H_2$-to-$O_2$ ratio of approximately 2:1 indicates that the produced $H_2$ originates from direct biophotolysis.

Next, the efficiency of $H_2$ production induced by anaerobiosis in HS medium (performed as described above) was compared with the classical sulphur deprivation method that works efficiently only in the presence of acetate (Fouchard et al., 2005; Kosourov et al., 2007). In anaerobically induced cultures kept in HS media about 20 $\mu l/ml$ $H_2$ was produced during the first 24 hours; in order to avoid overaccummulation of $H_2$ in the headspace of the cultures, the produced $H_2$ was removed every day by $N_2$ flushing. Using this method, the total $H_2$ production was about 65 $\mu l$ $H_2/ml$ culture in 96 hours. In the case of sulphur deprivation, $H_2$ production was minor during the first 24 hours, and the daily maximum amounts of $H_2$ were detected after 48 to 72 hours of sulphur deprivation; the total production was about 42 $\mu l$ $H_2/ml$ culture in 96 hours. This latter value is consistent when using sealed flasks and the same CC-124 strain (e.g. Steinbeck et al., 2015, Nagy et al., 2016), but it is below the yields obtained in improved photobioreactor systems (about 200 $\mu l$ $H_2/ml$ culture in 4 days, Kosourov et al., 2003).

About 40 $\mu l$ $O_2/mL$ culture accumulated in the headspace during 24 hours in the sulphur-deprived cultures (Fig. 1D, corresponding to about 1.3% $O_2$ in the gas phase), whereas only about 9 $\mu l/ml$ $O_2$ was found in the HS-cultures after 24 hours, which decreased further throughout the experiment.

*Origin and regulation of $H_2$ production in acetate-free media*

In order to unravel the mechanism of $H_2$ production in acetate-free media, the cells were treated with 3-(3',4'-dichlorophenyl)-1,1-dimethylurea (DCMU), which irreversibly binds to the $Q_B$ site in PSII. Upon the application of DCMU, $O_2$ concentration decreased and $H_2$ production was halted almost immediately (Figs. 2A and B), demonstrating the direct dependence of both on linear electron transport.

In Chlamydomonas, acetate assimilation may occur via the tricarboxylic acid cycle and the glyoxylate cycle, which are metabolically linked to gluconeogenesis and the oxidative pentose phosphate pathway (Johnson and Alric 2012, Plancke et al., 2014). The released $CO_2$ and glycerate 3-phosphate feeds the CBB cycle for which the reducing power generated by the photosynthetic electron transport is also utilized, as indicated by the extreme light sensitivity of various Rubisco mutants (Pinto et al., 2013). We hypothesized that in the absence of acetate and $CO_2$, the CBB cycle is inactive and the reducing power generated by the photosynthetic electron transport is more efficiently transferred to the hydrogenases. In order to ascertain about this, we added 2% $CO_2$ (v/v) into the headspace of the cultures. As a result, $H_2$ production dropped, whereas $O_2$ production strongly increased (Figs. 2C and 2D). When the CBB cycle inhibitor, glycolaldehyde was added, $H_2$ production and $O_2$ evolution were unchanged during the initial 6 h (Figs. 2E, 2F), indicating that the CBB cycle had been inactive. At a later time point, both $H_2$ production and $O_2$ evolution declined, most probably due a side-effect of glycolaldehyde. Thus the lack of acetate and $CO_2$ prevented the activation of the CBB cycle and electrons were more efficiently transferred to the hydrogenases.

Upon a low substrate availability of the CBB cycle, the consumption of ATP and NADPH is decreased, resulting in a strong thylakoid lumen acidification, which triggers photoprotective quenching mechanisms and slows down linear electron transport by "photosynthetic flux control" at the cytochrome $b_6f$ complex, resulting in a reduced PQ-pool (Kirchoff et al., 2000, Yamori et al., 2011, reviewed by Schöttler and Tóth 2014). Under these conditions, the rate of charge recombination in PSII increases and the rate of $O_2$ evolution decreases. The metabolic imbalance in the photosynthetic electron transport may be alleviated by alternative pathways in Chlamydomonas, including the Mehler reaction (Mehler, 1951), the plastid terminal oxidase and the flavodiiron-dependent photoreduction reduction pathways (reviewed by Erickson et al., 2015). However, all these pathways are $O_2$-dependent and under hypoxic conditions electron transport to the hydrogenases may represent the most efficient safety valve.

To test the possibility that thylakoid lumen acidification may limit linear electron transport and thereby $O_2$ and $H_2$ production, the cultures were treated with the ionophore carbonilcyanide p-triflouromethoxyphenylhydrazone (FCCP, Heytler 1962). Figures 2G and 2H show that upon the addition of FCCP, both $H_2$ and $O_2$ evolution temporarily increased.

When measuring the rate of $H_2$ production with a relatively high time resolution following anaerobic induction (Fig. 3A), we observed an initial burst of $H_2$ production (about 2.3 $\mu$l $H_2$/ml culture was produced in 8 min, corresponding to a rate of 15.76 $\mu$mole $H_2$ mg$^{-1}$ Chl h$^{-1}$), followed by a slower $H_2$ production rate (1.31 $\mu$mole $H_2$ mg$^{-1}$ Chl h$^{-1}$ between 60 to 300 min), during which about 6.2 $\mu$l $O_2$/ml culture, equaling 0.2% $O_2$, accumulated in the gas phase (Fig. 3B). The hydrogenases of *Chlamydomonas reinhardtii* have an $I_{50}$ of 0.3-0.4 % $O_2$ as determined upon a 2-min incubation *in vivo* (Ghirardi et al., 1997), thus prolonged exposure to 0.2% $O_2$ is likely to be inhibitory to the hydrogenases.

A perspective way to keep the hydrogenases active may be eliminating the produced $O_2$. As a first approach, we added glucose (Glu), glucose oxidase (GO) and ascorbate (Asc) to the cultures to efficiently scavenge $O_2$ (Joët et al., 2002). Fig. 3D shows that upon this treatment, the amount of $O_2$ in the headspace was reduced by half in the first hour, and concomitantly, the amount of $H_2$ produced in 3 hour increased from about 8 to 80 $\mu$l $H_2$/ml culture (Fig. 3C). These values correspond to light-to-$H_2$ energy conversion efficiencies of 0.43 % and 4.45% in the absence and presence of Glu+GO+Asc, respectively (Table 1). However, the application of Glu+GO+Asc is not a viable option for a long-term $H_2$ production thus we searched for another possibility.

**Example 2** - **Preserving hydrogenase activity using an iron-salt based $O_2$ absorbent**

Decreasing the amount of $O_2$ in the cultures can be achieved by i) downregulating PSII activity (e.g. Melis et al., 2000, Surzyczki et al., 2007), which is precarious, because PSII is the main source of reducing power; ii) increasing the respiration:photosynthesis ratio (Scoma et al., 2014), which has the disadvantage that it has a low energy conversion efficiency and requires significant amounts of organic substrates; and iii) bacterial respiration (Lakatos et al., 2014), with the drawback that it also requires acetate or other organic carbon source; in addition, hemoglobin, myoglobin and cobalt chelates have been also used in short-term experiments to remove the evolved $O_2$ (Rosenkrans and Krasna, 1984), or a very intense flushing with inert gases (Reeves and Greenbaum, 1985)).

Here, as chemical $O_2$ absorbent, a mixture of iron powder and sodium chloride, widely used in the food industry, was applied. It is highly efficient (1.5 g catalyst can absorb up to 20 ml $O_2$ at room temperature; e.g. Braga et al., 2010), biologically safe and very cheap. A small amount (approximately 1.3 g) of $O_2$ absorbent was placed into a 2 ml vial and introduced into the headspace of the serum bottle, above the Chlamydomonas culture (Fig. 4A). Using this system, the $O_2$ concentration was diminished to about 2.1 $\mu$l $O_2$ ml$^{-1}$ culture (Fig. 4C), corresponding to about 0.07% $O_2$ in the gas phase. The decrease in $O_2$ concentration was accompanied by an about two-fold increase in $H_2$ production on the timescale of 96 hours (Fig. 4B), reaching about 200 $\mu$l $H_2$ ml$^{-1}$ culture. At the beginning of the experiment, the light to $H_2$ energy conversion efficiency was about 2.3% both in the absence and presence of the $O_2$ absorbent; during prolonged illumination, energy conversion efficiency was better maintained when the $O_2$ absorbent was present (Table 1). It is to be noted that in sulphur deprivation experiments carried out in photobioreactors and in the presence of acetate, the maximum light to $H_2$ conversion efficiency was in the range of 1.5 %, whereas in nutrient-replete conditions with acetate, it was much lower, about 0.1% in a PsbA protein mutant strain (Scoma et al., 2014).

Next, the expression and the activity of hydrogenase in the presence or absence of the $O_2$ absorbent were assessed. During the 4-h dark anaerobic induction period, the *HYDA1* transcript level increased about 11-fold as determined by quantitative RT-PCR analysis (Fig. 5A), which was followed by a decrease both in the presence and absence of the $O_2$ absorbent. The amount of HydA enzyme, as determined semi-quantitatively by western blot analysis, also showed a strong upregulation upon the 4-hour dark anaerobic induction period. Later on, the amount of HydA decreased, which was attenuated by the $O_2$ absorbent (Fig. 5B). Fig. 5C shows that upon a prolonged illumination in the presence of the $O_2$ absorbent, approximately 2.5 times higher *in vitro* hydrogenase activity remained relative to the control.

### Example 3 - Photosynthetic activity and sustainability

In order to gain information about the sustainability of our system, we characterized the photosynthetic apparatus during the 96-hour $H_2$ producing period. The chl(a+b) content decreased only by a few percent during the 96-hour experiment (Fig. 6A). The fast chl *a* fluorescence (OJIP) transient, a sensitive and widely used indicator of photosynthetic function (e.g. Schansker et al., 2014), decreased moderately in intensity (Fig. 6B). The $F_v/F_M$ value, an indicator of PSII efficiency slowly decreased during the 96-h $H_2$ producing period, but remained relatively high (above 0.4) both in the presence and absence of the $O_2$ absorbent (Fig. 6C).

The amounts of PsbA (reaction center protein of PSII), PetB (a subunit of the cytochrome $b_6/f$ complex) and PsaA (reaction center protein of PSI) remained largely unaltered both in presence and absence of the $O_2$ absorbent as determined by western blot analysis (Fig. 6D). These findings are in strong contrast to sulphur deprivation in TAP media, where the PSII reaction center protein PsbA is diminished within 2-to-3 days and by the end of a 4-to-6 day period of sulphur deprivation, most photosynthetic complexes are degraded and the cells may eventually die (Melis et al., 2000, Zhang et al., 2002, Nagy et al., 2016).

Based on the finding that the cultures remain photosynthetically active, it was attempted to recover and reuse the cultures after the $H_2$-producing period; to this end, the cultures were transferred into a multi-well cultivation instrument and the cultures were bubbled with 1% $CO_2$ in HS medium for 72 hours. The algal cultures showed vigorous growth (Fig. 7A); when the cells were subjected to second anaerobic induction period, efficient $H_2$ production was observed again (Figs. 7B and C).

### Example 4 - Algal growth conditions and $H_2$ production

*C. reinhardtii* CC-124 strain was grown initially in Tris-acetate-phosphate (TAP) medium at a light intensity of 80-90 $\mu$mole photons $m^{-2}$ $s^{-1}$ and at 22 °C in an algal growth chamber. After three days of cultivation, the cells were transferred to high-salt (HS) medium, HS supplemented with acetate (HSA), Tris-phosphate (TP) or TAP media (http://www.chlamycollection.org/methods/media-recipes/) and the chl content was set at 50 $\mu$g chl(a+b)/ml (Porra et al., 1989). For $H_2$ production, 30 ml culture was placed in 120 ml serum bottles and sealed off with rubber septa under sterile conditions (Fig. 4A). For anaerobiosis treatment, the gas phase of the bottle was flushed with $N_2$ gas for 10 min and kept in the dark for 4 hours, during which the vials were flushed twice more with $N_2$. Following this, the cultures were placed in continuous white light (approximately 320 $\mu$mole photons $m^{-2}$ $s^{-1}$), at 26-28° C for 96 hours.

In the regeneration experiment following $H_2$ production, a Multi-Cultivator MC 1000-OD instrument (Photon Systems Instruments, Brno, Czech Republic) was used. At the start of cultivation, the chl content was set at 6 $\mu$g chl(a+b)/ml in HS medium. The cells were grown at 23 ° C, 80 $\mu$mole photons $m^{-2}$ $s^{-1}$ in continuous light for 72 hours and the cultures were bubbled with air containing 1% $CO_2$. Following this, the cultures were subjected to a second round of anaerobic induction in HS medium, as described above.

### Example 5 - $H_2$ Production

Alga cultures containing 50 $\mu$g chl(a+b)/ml were illuminated at a light intensity of 32 W/$m^2$, corresponding to approximately 320 $\mu$mole photons $m^{-2}s^{-1}$, for 24 hours. Using the measured incident and the absorbed light energy (in parentheses) and the rate of $H_2$ production, the light energy conversion efficiency was calculated. The experiments were carried out in HS medium (control) and the effects of a combined glucose (Glu), glucose oxidase (GO) and ascorbate (Asc) treatment and the effects of an iron-salt $O_2$ absorbent were also assessed. n.d., non-determined.

**Table 1.** Efficiency of light to $H_2$ energy conversion in *Chlamydomonas reinhardtii* in HS medium under various conditions, as determined as a percentage of the measured incident and the absorbed light energy (in parentheses). The experiments were carried out in HS medium (control) and the effects of a combined glucose (Glu, 2 mM), glucose oxidase (GO, 0.2 mg/ml) and ascorbate (Asc, 1 mM) treatment and the effects of an iron-salt $O_2$ absorbent were also assessed; n.d., non-determined.

| Conditions of $H_2$ production | Time of illumination following a 4-h anaerobic induction (h) | | | | |
|---|---|---|---|---|---|
| | 0.25 | 1 | 3 | 5 | 24 |
| Control (HS) | 2.23 % (2.53 %) | 0.60 % (0.68 %) | 0.43 % (0.48 %) | 0.36 % (0.41 %) | 0.19 % (0.22 %) |
| +Glu+Glu oxidase+Asc | n.d. | 6.03 % (6.85 %) | 4.45 % (5.05 %) | 2.90 % (3.29 %) | 0.76 % (0.86 %) |

(continued)

| Conditions of $H_2$ production | Time of illumination following a 4-h anaerobic induction (h) | | | | |
|---|---|---|---|---|---|
| | 0.25 | 1 | 3 | 5 | 24 |
| +$O_2$ absorbent | 2.29 % (2.60 %) | n.d. | 0.82 % (0.94 %) | 0.72 % (0.82 %) | 0.54 % (0.61 %) |

**Example 6** - **Further alga species and strains used for $H_2$ productions**

The invention was demonstrated to work on two more strains of C. *reinhardtii,* called CC-409 and the *tla3 (truncated light-harvesting antenna size3)* mutant (see Fig. 8). The pre-cultures were grown the same way as in the case of the CC-124 strain. The inventive method worked also on two *Chlorella* species, called *Chlorella sorokiniana,* strain 211-32 and *Chlorella vulgaris,* strain 15-2075 (see Fig. 8). *Chlorella* pre-cultures were grown in HS medium for six days.

**DISCUSSION**

The potential energy conversion efficiency from sunlight to $H_2$ by green algae is in the range of 10 to 13 %; however, in nature, it lasts only for a few minutes due to the inhibition of hydrogenases by the evolved $O_2$ (Hallenbeck and Benemann, 2002; Dubini and Ghirardi 2015).

In the present invention a fully photoautotrophic, efficient and sustainable $H_2$ production by *Chlamydomonas reinhardtii* is established, which is based on keeping the CBB cycle inactive by substrate limitation; in order to better preserve hydrogenase activity, the evolved $O_2$ may be removed. The inventive system has fundamental advantages relative to the earlier methods, namely that:

i. $H_2$ production starts promptly upon illumination,
ii. it does not require sulphur deprivation,
iii. it does not require acetate, thus it is fully photoautotrophic,
iv. because no organic carbon source is required, the risk of bacterial contamination would be low,
v. the cultures remain photosynthetically active during the $H_2$ production phase and they can be efficiently recovered afterwards,
vi. it is based entirely on direct biophotolysis, and has relatively high light energy conversion efficiency,
vii. during the growth phases, $CO_2$, as an industrial by-product can be efficiently utilized, and
viii. it can make use of relatively high light intensities.

The maximum $H_2$ production yield achieved using this protocol was about 200 $\mu$l $H_2$/ml culture in 96 hours, which is approximately four times higher as observed here for sulphur-deprived cultures (compare Figs. 1C and 4A), and it is in the same range as the maximum observed earlier using an advanced photobioreactor in the case of sulphur-deprived cultures (Kosourov et al., 2003). It is expected that the yield of $H_2$ production will be further improved using photobioreactors with even light conditions (e.g. Gianelli et al., 2009), and by applying the inventive method to various photosynthetic mutants possessing e.g. truncated light-harvesting antennae (Kosorouv et al., 2011), or a high PsbA protein content (Scoma et al., 2012), or mutants possessing hydrogenases with decreased $O_2$ sensitivity.

Keeping the CBB cycle inactive can be achieved by substrate limitation; another key factor to reach a sustained $H_2$ production is to protect the hydrogenases from $O_2$. Therefore an iron-salt-based $O_2$ absorbent was applied and the $O_2$ concentration in the headspace decreased below 0.1%. In the future, more advanced materials, based on reversible $O_2$ chemisorption may become available, for instance, crystalline salts of cationic multimetallic cobalt complexes (Sundberg et al., 2014). Ideally, the $O_2$ scavenger would be introduced directly into the liquid phase for a prompt effect. By further decreasing the $O_2$ level, it is expected that hydrogenase activity will be better preserved and it could act as an efficient electron sink; as a result, lumen acidification and photosynthetic flux control will be attenuated, and the yield of $H_2$ production will be further increased. In summary, the present results demonstrate the usability of algal cells as whole-cell catalysts for $H_2$ production.

REFERENCES

Antal TK, Krendeleva TE, Tyystjärvi E (2015) Multiple regulatory mechanisms in the chloroplast of green algae: relation to hydrogen production. Photosynth Res 125: 357-381

Braga LR, Sarantopoulos CIGL, Peres L, Braga JWB (2010) Evaluation of absorption kinetics of oxygen scavenger sachets using response surface methodology. Packag. Technol. Sci. 23: 351-361

Chochois V, Dauvillée D, Beyly A, Tolleter D, Cuine S, Timpano H, Ball S, Cournac L, Peltier G (2009) Hydrogen production in Chlamydomonas: photosystem II-dependent and -independent pathways differ in their requirement for starch metabolism. Plant Physiol. 151: 631-40

Cinco RM, Macinnis JM, Greenbaum E (1993) The role of carbon dioxide in light-activated hydrogen production by Chlamydomonas reinhardtii. Photosynth Res. 38): 27-33

Degrenne B, Pruvost J, Legrand J (2011) Effect of prolonged hypoxia in autotrophic conditions in the hydrogen production by the green microalga Chlamydomonas reinhardtii in photobioreactor. Bioresour. Technol.102: 1035-43

Dubini A, Ghirardi ML (2015) Engineering photosynthetic organisms for the production of biohydrogen. Photosynth Res 123: 241-253

Eivazova ER, Markov SA (2012) Conformational regulation of the hydrogenase gene expression in green alga Chlamydomonas reinhardtii. Int J Hydrogen Energy 37: 17788-17793

Erickson E, Wakao E, Niyogi KK (2015) Light stress and photoprotection in Chlamydomonas reinhardtii. Plant J 82: 449-465

Fouchard S, Hemschemeier A, Caruana A, Pruvost J, Legrand J, Happe T, Peltier G, Cournac L (2005) Autotrophic and mixotrophic hydrogen photoproduction in sulfur-deprived Chlamydomonas cells. Appl Environ Microbiol 71: 6199-6205

Ghirardi ML, Togasaki RK, Seibert M (1997) Oxygen sensitivity of algal H2-production. Appl Biochem Biotechnol 63: 141-151

Godaux D, Bailleul B, Berne N, Cardol P (2016) Induction of photosynthetic carbon fixation in anoxia relies on hydrogenase activity and PGRL1-mediated cyclic electron flow in Chlamydomonas reinhardtii. Plant Physiology 168: 648-658

Hallenbeck PC, Benemann JR (2002) Biological hydrogen production; fundamentals and limiting processes. Int J Hydrogen Energy 27: 1185-1193

Harris EH (2009) The Chlamydomonas Sourcebook: Introduction to Chlamydomonas and its laboratory use. Academic Press, San Diego, CA

Hemschemeier A, Melis A, Happe T (2009) Analytical approaches to photobiological hydrogen production in unicellular green algae. Photosynth Res 102: 523-540

Heytler, PG (1962) "A new class of uncoupling agents - Carbonyl cyanide phenylhydrazones". Biochemical and Biophysical Research Communications. 7: 272-275

Joët T, Genty B, Josse E-M, Kuntz M, Cournac L, Peltier G (2002) Involvement of a plastid terminal oxidase in plastoquinone oxidation as evidenced by expression of the Arabidopsis thaliana enzyme in tobacco. 277: 31623-31630

Johnson X, Alric J (2012) Interaction between starch breakdown, acetate assimilation, and photosynthetic cyclic electron flow in Chlamydomonas reinhardtii J Biol Chem 287: 26445-26452

Jurado-Oller JL, Dubini A, Galvan A, Fernández E, Gonzalez-Ballester D (2015) Low oxygen levels contribute to improve photohydrogen production in mixotrophic non-stressed Chlamydomonas cultures. Biotechnol Biofuels 8: 149

Kosourov S, Patrusheva E, Ghirardi ML, Seibert M, Tsygankov A (2007) A comparison of hydrogen photoproduction by sulfur-deprived Chlamydomonas reinhardtii under different growth conditions. J Biotechnol 128: 776-787

Kosourov S, Seibert M, Ghirardi ML (2003) Effects of extracellular pH on the metabolic pathways in sulfur-deprived, H2-producing Chlamydomonas reinhardtii cultures. Plant Cell Physiol 44: 146-155

Kosourov SN, Seibert M (2009) Hydrogen photoproduction by nutrient-deprived Chlamydomonas reinhardtii cells immobilized within thin alginate films under aerobic and anaerobic conditions. Biotechnol Bioeng 102: 50-58

Lakatos G, Deak Z, Vass I, Rétfalvi T, Rozgonyi S, Rákhely G, Ördög V, Kondorosi E, Maróti G (2014) Bacterial symbionts enhance photo-fermentative hydrogen evolution of Chlamydomonas algae. Green Chemistry 16: 4716-4727

Leliaert F, Verbruggen H, Zechman W (2011) Into the deep: New discoveries at the base of the green plant phylogeny. Bioessays 33: 683-692

Marin B (2012) Nested in the Chlorellales or independent class? Phylogeny and classification of the Pedinophyceae (Viridiplantae) revealed by molecular phylogenetic analyses of complete nuclear and plastid-encoded rRNA operons. Protist. 163: 778-805

Mehler A (1951) Studies on the reactions of illuminated chloroplasts. II. Stimulation on inhibition of the reaction with molecular oxygen. Arch Biochem Biophys 34. 339-351

Melis A, Zhang L, Forestier M, Ghirardi ML, Seibert M (2000) Sustained photobiological hydrogen gas production upon reversible inactivation of oxygen evolution in the green alga Chlamydomonas reinhardtii. Plant Physiol 122:

Nagy V, Vidal-Meireles A, Tengölics R, Rákhely G, Garab G, Kovacs L, Tóth SZ (2016) Ascorbate accumulation during sulphur deprivation and its effects on photosystem II activity and H2 production of the green alga Chlamydomonas reinhardtii Plant Cell Environ 39: 1460-1472

Oncel S, Kose A (2014) Comparison of tubular and panel type photobioreactors for biohydrogen production utilizing Chlamydomonas reinhardtii considering mixing time and light intensity. Biores Technol 151: 265-270

Pinto TS, Malcata FX, Arrabaça JD, Silva JM, Spreitzer RJ, Esquivel MG (2013) Rubisco mutants of Chlamydomonas reinhardtii enhance photosynthetic hydrogen production. Appl Microbiol Biotechnol 97: 5635-5643

Plancke C, Vigeolas H, Höhner R, Roberty S, Emonds-Alt B, Larosa V, Willamme R, Duby F, Onga Dhali D, Thonart P, Hiligsmann S, Franck F, Eppe G, Cardol P, Hippler M, Remacle C (2014) Lack of isocitrate lyase in Chlamydomonas leads to changes in carbon metabolism and in the response to oxidative stress under mixotrophic growth. Plant J 77: 404-417

Porra RJ, Thompson WA, Kriedeman PE (1989) Determination of accurate extinction coefficients and simultaneous equations for essaying chlorophylls-a and -b with four different solvents: verification of the concentration of chlorophyll standards by atomic absorption spectroscopy. Biochim Biophys Acta 975: 384-394

Reeves M, Greenbaum E (1985) Long-term endurance and selection studies in hydrogen and oxygen photoproduction by Chlamydomonas reinhardtii. Enzyme Microb Technol 7: 169-174

Rosenkrans AM, Krasna AI (1984) Stimulation of hydrogen photoproduction in algae by removal of oxygen by reagents that combine reversibly with oxygen. Biotechnol Bioeng 26: 1334-1342

Schansker G, Tóth SZ, Holzwarth AR, Garab G (2014) Chlorophyll a fluorescence: beyond the limits of the QA model. Photosynthesis Research 120: 43-58

Schöttler MA, Tóth SZ (2014) Photosynthetic complex stoichiometry dynamics in higher plants: environmental acclimation and photosynthetic flux control. Frontiers Plant Sci 5: 188

Scoma A, Durante L, Bertin L, Fava F (2014) Acclimation to hypoxia in Chlamydomonas reinhardtii Can biophotolysis be the major trigger for long-term H2 production? New Phytol. 204: 890-900

Scoma A, Krawietz D, Faraloni C, Giannelli L, Happe T, Torzillo G (2012) Sustained H2 production in a Chlamydomonas reinhardtii D1 protein mutant. J Biotechnol 157: 613-619

Scoma A, Tóth SZ (2017) On the pathways feeding the H2 production process in nutrient-replete, hypoxic conditions. Commentary on the article "Low oxygen levels contribute to improve photohydrogen production in mixotrophic non-stressed Chlamydomonas cultures", by Jurado-Oller et al., Biotechnology for Biofuels, published September 7, 2015; 8:149. Biotechnol Biofuels 10: 116

Steinbeck J, Nikolova D, Weingarten R, Johnson X, Richaud P, Peltier G, Hermann M, Magneschi L, Hippler M. (2015) Deletion of Proton Gradient Regulation 5 (PGR5) and PGR5-Like 1 (PGRL1) proteins promote sustainable light-driven hydrogen production in Chlamydomonas reinhardtii due to increased PSII activity under sulfur deprivation. Front Plant Sci 6:892

Sundberg J, Cameron LJ, Southon PD, Kepert CJ, McKenzie CJ (2014) Oxygen chemisorption/desorption in a reversible single-crystal-to-single-crystal transformation. Chem Sci 5: 4017-4025

Surzycki R, Cournac L, Peltier G, Rochaix J-D (2007) Potential for hydrogen production with inducible chloroplast gene expression in Chlamydomonas. Proc Nat Acad Sci USA 104: 17548-17553

Swanson KD, Ratzloff MW, Mulder DW, Artz JH, Ghose S, Hoffman A, White S, Zadvornyy OA, Broderick JB, Bothner B, King PW, Peters JW (2015) [FeFe]-hydrogenase oxygen inactivation is initiated at the H Cluster 2Fe subcluster. J Am Chem Soc 137, 1809-1816.

Vidal-Meireles A, Neupert J, Zsigmond L, Rosado-Souza L, Kovacs L, Nagy V, Galambos A, Fernie AR, Bock R, Tóth SZ (2017) Regulation of ascorbate biosynthesis in green algae has evolved to enable rapid stress-induced response the VTC2 gene encoding GDP-L-galactose phosphorylase. New Phytologist (in press), DOI: 10.1111/nph.14425

Yamori W, Takahashi S, Makino A, Price D, Badger MR, von Caemmerer S (2011) The roles of ATP synthase and the cytochrome b6/f complexes in limiting chloroplast electron transport and determining photosynthetic capacity. Plant Physiol 155: 956-962

Zhang L., Happe T. & Melis A. (2002) Biochemical and morphological characterization of sulfur-deprived and H2-producing Chlamydomonas reinhardtii (green alga). Planta 214, 552-561.

## Claims

1. A method of hydrogen gas generation, comprising the steps of:

    i. culturing under anaerobic conditions in the dark a green alga species selected from the group consisting of *Chlamydomonas sp., Chlorella sp.,* or *Scenedesmus sp* which is capable of expressing one or more types of hydrogenase enzymes (induction phase);

ii. subsequently culturing the green alga under illuminated conditions in a medium substantially devoid of organic or inorganic carbon sources (production phase), whereupon activation of the CBB cycle in the alga is prevented, wherein substantially devoid of organic or inorganic carbon sources refers to a $CO_2$ content of less than 0.03% in the headspace of the culture, and wherein the light intensity is between 15 - 3100 $\mu$mol photons $m^{-2}s^{-1}$

iii. removing the produced oxygen, and

iv. collecting the evolved hydrogen gas.

2. The method of claim 1, wherein the green alga is capable of expressing hydrogenase enzymes in the chloroplast.

3. The method of claim 1 or 2, wherein the alga is *Chlamydomonas reinhardtii, Chlorella sorokiniana,* or *Chlorella vulgaris.*

4. The method of claim 3, wherein the *Chlamydomonas reinhardtii* strain is CC-124, CC-409, or *tla3,* the *Chlorella sorokiniana* strain is *211-32,* and the *Chlorella vulgaris* strain is *15-2075.*

5. The method according to any one of claims 1 to 4, wherein $O_2$ is removed mechanically, chemically or biologically.

6. The method according to claim 5, wherein $O_2$ is removed chemically by iron-based oxygen absorbent, a cobalt complex, a copper-, palladium- or alumina containing catalysts.

7. The method according to any one of claims 1 to 6, wherein the medium of step ii) is limited in any organic or inorganic carbon source during the $H_2$ production phase.

8. The method according to any one of claims 1 to 7, wherein no $CO_2$, carbonate or acetate is added to the medium during the $H_2$ production phase of step ii).

9. The method according to any one of claims 1 to 8, wherein the medium of step ii) is a tris-phosphate or a high-salt medium.

10. The method according to any one of claims 1 to 9, wherein the headspace of the culture contains less than 300 ppm $CO_2$, or less than 30 ppm, or less than 3 ppm.

11. The method according to any of claims 1 to 10, wherein the culture is illuminated by solar radiation or by an artificial light source.

12. The method according to any one of claims 1 to 11, wherein thereby at least 0.5 mL/h L hydrogen are produced.

13. The method according to any one of claims 1 to 12, wherein hydrogenase enzyme expression in the alga is increased in the induction phase due to anoxia.

14. The method according to any one of claims 1 to 13, wherein the alga biomass is regenerated after hydrogen production.

**Patentansprüche**

1. Verfahren zur Erzeugung von Wasserstoffgas, umfassend die folgenden Schritte:

i. Kultivieren einer Grünalge unter anaeroben Bedingungen im Dunkeln, wobei die Grünalge ausgewählt ist aus der Gruppe bestehend aus *Chlamydomonas sp., Chlorella sp.,* und *Scenedesmus sp,* und die zur Expression eines oder mehrerer Typen von Hydrogenase-Enzymen befähigt ist (Induktionsphase);

ii. danach Kultivieren der Grünalge unter beleuchteten Bedingungen in einem Medium, das im Wesentlichen frei von organischen oder anorganischen Kohlenstoffquellen ist (Produktionsphase), woraufhin die Aktivierung des CBB-Zyklus in der Alge verhindert ist; wobei sich im Wesentlichen frei von organischen oder anorganischen Kohlenstoffquellen ein $CO_2$-Gehalt von weniger als 0,03% im Kopfraum der Kultur bezieht, und wobei die Lichtintensität zwischen 15 - 3100 $\mu$mol Photonen $m^{-2}s^{-1}$ liegt;

iii. Entfernen des erzeugten Sauerstoffs, und
iv. Sammeln des entstandenen Wasserstoffgases.

2. Verfahren nach Anspruch 1, wobei die Grünalge zur Expression von Hydrogenase-Enzymen im Chloroplasten befähigt ist.

3. Verfahren nach Anspruch 1 oder 2, wobei die Alge *Chlamydomonas reinhardtii, Chlorella sorokiniana* oder *Chlorella vulgaris* ist.

4. Verfahren nach Anspruch 3, wobei der *Chlamydomonas reinhardtii*-Stamm CC-124, CC-409 oder *tla3,* der *Chlorella sorokiniana* Stamm *211-32,* und der *Chlorella vulgaris* Stamm *15-2075* ist.

5. Verfahren nach einem der Ansprüche 1 bis 4, wobei $O_2$ mechanisch, chemisch oder biologisch entfernt wird.

6. Verfahren nach Anspruch 5, wobei $O_2$ durch ein eisenbasiertes Sauerstoffabsorbens, einen Kobaltkomplex, einen Kupfer-, Palladium- oder Aluminiumoxid-haltigen Katalysator chemisch entfernt wird.

7. Verfahren nach einem der Ansprüche 1 bis 6, wobei das Medium aus Schritt ii) während der $H_2$-Produktionsphase in der organische oder anorganische Kohlenstoffquelle beschränkt ist.

8. Verfahren nach einem der Ansprüche 1 bis 7, wobei das Medium aus Schritt ii) nicht $CO_2$, Carbonat oder Acetat enthält.

9. Verfahren nach einem der Ansprüche 1 bis 8, wobei das Medium aus Schritt ii) ein Tris-Phosphat oder ein Hochsalzmedium ist.

10. Verfahren nach einem der Ansprüche 1 bis 9, wobei der Kopfraum der Kultur weniger als 300 ppm $CO_2$, oder weniger als 30 ppm, oder weniger als 3 ppm, enthält.

11. Verfahren nach einem der Ansprüche 1 bis 10, wobei die Kultur durch Sonneneinstrahlung oder durch eine künstliche Lichtquelle beleuchtet wird.

12. Verfahren nach einem der Ansprüche 1 bis 11, wobei mindestens 0,5 mL/h an Wasserstoff erzeugt werden.

13. Verfahren nach einem der Ansprüche 1 bis 12, wobei die Expression von Hydrogenase-Enzym in der Alge in der Induktionsphase aufgrund von Anoxie erhöht wird.

14. Verfahren nach einem der Ansprüche 1 bis 13, wobei die Algenbiomasse nach der Wasserstofferzeugung regeneriert wird.

**Revendications**

1. Procédé de génération d'hydrogène gazeux, comprenant les étapes consistant à :

i. cultiver dans l'obscurité d'une espèce une algue verte dans des conditions anaérobies, choisie dans le groupe constitué par *Chlamydomonas sp., Chlorella sp.,* ou *Scenedesmus sp.,* qui est capable d'exprimer un ou plusieurs types d'enzymes hydrogénases (phase d'induction) ;
ii. cultiver ensuite l'algue dans des conditions éclairées dans un milieu essentiellement dépourvu de sources de carbone organiques ou inorganiques (phase de production), ce qui empêche l'activation du cycle CBB dans l'algue, dans lequel substantiellement dépourvue de sources de carbone organique ou inorganique se réfère à une teneur en $_{CO2}$ inférieure à 0,03% dans l'espace de tête de la culture, et dans lequel l'intensité lumineuse est comprise entre 15 et 3100 photons $\mu$mol m$^{-2}$s$^{-1}$ ;
iii. éliminer l'oxygène produit, et
iv. recueillir l'hydrogène gazeux dégagé.

2. Procédé selon la revendication 1, dans lequel l'algue verte est capable d'exprimer des enzymes hydrogénases dans le chloroplaste.

3. Procédé selon la revendication 1 ou 2, dans lequel l'algue est *Chlamydomonas reinhardtii, Chlorella sorokiniana* ou *Chlorella vulgaris.*

4. Procédé selon la revendication 3, dans lequel la souche *Chlamydomonas reinhardtii* est CC-124, CC-409 ou *tla3,* la souche *Chlorella sorokiniana* est 211-32, et la souche *Chlorella vulgaris* est 15-2075.

5. Procédé selon l'une quelconque des revendications 1 à 4, dans lequel l'$O_2$ est éliminé mécaniquement, chimiquement ou biologiquement.

6. Procédé selon la revendication 5, dans lequel l'$O_2$ est éliminé chimiquement par un absorbant d'oxygène à base de fer, un complexe de cobalt, des catalyseurs contenant du cuivre, du palladium ou de l'alumine.

7. Procédé selon l'une quelconque des revendications 1 à 6, dans lequel le milieu de l'étape ii) est limité in toute source de carbone organique ou inorganique.

8. Procédé selon l'une quelconque des revendications 1 à 7, dans lequel le milieu de l'étape ii) ne contient pas de $CO_2$, de carbonate ou d'acétate.

9. Procédé selon l'une quelconque des revendications 1 à 8, dans lequel le milieu de l'étape ii) est un tris-phosphate ou un milieu riche en sel.

10. Procédé selon l'une quelconque des revendications 1 à 9, dans lequel l'espace de tête de la culture contient moins de 300 ppm de $CO_2$, ou moins de 30 ppm, ou moins de 3 ppm.

11. Procédé selon l'une quelconque des revendications 1 à 10, dans lequel la culture est éclairée par un rayonnement solaire ou par une source de lumière artificielle.

12. Procédé selon l'une quelconque des revendications 1 à 11, dans lequel au moins 0,5 mL/h d'hydrogène sont produits.

13. Procédé selon l'une quelconque des revendications 1 à 12, dans lequel l'expression de l'enzyme hydrogénase dans l'algue est augmentée dans la phase d'induction due à l'anoxie.

14. Procédé selon l'une quelconque des revendications 1 à 13, dans lequel la biomasse de l'algue est régénérée après production d'hydrogène.

Fig. 1

Fig. 2

Fig. 3

Fig. 4

A

B

C

Fig. 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100273149 A
- US 20090221052 A

### Non-patent literature cited in the description

- **ANTAL TK ; KRENDELEVA TE ; TYYSTJÄRVI E.** Multiple regulatory mechanisms in the chloroplast of green algae: relation to hydrogen production. *Photosynth Res,* 2015, vol. 125, 357-381
- **BRAGA LR ; SARANTOPOULOS CIGL ; PERES L ; BRAGA JWB.** Evaluation of absorption kinetics of oxygen scavenger sachets using response surface methodology. *Packag. Technol. Sci.,* 2010, vol. 23, 351-361
- **CHOCHOIS V ; DAUVILLÉE D ; BEYLY A ; TOLLETER D ; CUINE S ; TIMPANO H ; BALL S ; COURNAC L ; PELTIER G.** Hydrogen production in Chlamydomonas: photosystem II-dependent and -independent pathways differ in their requirement for starch metabolism. *Plant Physiol.,* 2009, vol. 151, 631-40
- **CINCO RM ; MACINNIS JM ; GREENBAUM E.** The role of carbon dioxide in light-activated hydrogen production by Chlamydomonas reinhardtii. *Photosynth Res.,* 1993, vol. 38, 27-33
- **DEGRENNE B ; PRUVOST J ; LEGRAND J.** Effect of prolonged hypoxia in autotrophic conditions in the hydrogen production by the green microalga Chlamydomonas reinhardtii in photobioreactor. *Bioresour,* 2011, vol. 102, 1035-43
- **DUBINI A ; GHIRARDI ML.** Engineering photosynthetic organisms for the production of biohydrogen. *Photosynth Res,* 2015, vol. 123, 241-253
- **EIVAZOVA ER ; MARKOV SA.** Conformational regulation of the hydrogenase gene expression in green alga Chlamydomonas reinhardtii. *Int J Hydrogen Energy,* 2012, vol. 37, 17788-17793
- **ERICKSON E ; WAKAO E ; NIYOGI KK.** Light stress and photoprotection in Chlamydomonas reinhardtii. *Plant J,* 2015, vol. 82, 449-465
- **FOUCHARD S ; HEMSCHEMEIER A ; CARUANA A ; PRUVOST J ; LEGRAND J ; HAPPE T ; PELTIER G ; COURNAC L.** Autotrophic and mixotrophic hydrogen photoproduction in sulfur-deprived Chlamydomonas cells. *Appl Environ Microbiol,* 2005, vol. 71, 6199-6205
- **GHIRARDI ML ; TOGASAKI RK ; SEIBERT M.** Oxygen sensitivity of algal H2-production. *Appl Biochem Biotechnol,* 1997, vol. 63, 141-151
- **GODAUX D ; BAILLEUL B ; BERNE N ; CARDOL P.** Induction of photosynthetic carbon fixation in anoxia relies on hydrogenase activity and PGRL1-mediated cyclic electron flow in Chlamydomonas reinhardtii. *Plant Physiology,* 2016, vol. 168, 648-658
- **HALLENBECK PC ; BENEMANN JR.** Biological hydrogen production; fundamentals and limiting processes. *Int J Hydrogen Energy,* 2002, vol. 27, 1185-1193
- **HARRIS EH.** The Chlamydomonas Sourcebook: Introduction to Chlamydomonas and its laboratory use. Academic Press, 2009
- **HEMSCHEMEIER A ; MELIS A ; HAPPE T.** Analytical approaches to photobiological hydrogen production in unicellular green algae. *Photosynth Res,* 2009, vol. 102, 523-540
- **HEYTLER, PG.** A new class of uncoupling agents - Carbonyl cyanide phenylhydrazones. *Biochemical and Biophysical Research Communications,* 1962, vol. 7, 272-275
- **JOËT T ; GENTY B ; JOSSE E-M ; KUNTZ M ; COURNAC L ; PELTIER G.** *Involvement of a plastid terminal oxidase in plastoquinone oxidation as evidenced by expression of the Arabidopsis thaliana enzyme in tobacco,* 2002, vol. 277, 31623-31630
- **JOHNSON X ; ALRIC J.** Interaction between starch breakdown, acetate assimilation, and photosynthetic cyclic electron flow. *Chlamydomonas reinhardtii J Biol Chem,* 2012, vol. 287, 26445-26452
- **JURADO-OLLER JL ; DUBINI A ; GALVÁN A ; FERNÁNDEZ E ; GONZALEZ-BALLESTER D.** Low oxygen levels contribute to improve photohydrogen production in mixotrophic non-stressed Chlamydomonas cultures. *Biotechnol Biofuels,* 2015, vol. 8, 149
- **KOSOUROV S ; PATRUSHEVA E ; GHIRARDI ML ; SEIBERT M ; TSYGANKOV A.** A comparison of hydrogen photoproduction by sulfur-deprived Chlamydomonas reinhardtii under different growth conditions. *J Biotechnol,* 2007, vol. 128, 776-787

- **KOSOUROV S ; SEIBERT M ; GHIRARDI ML.** Effects of extracellular pH on the metabolic pathways in sulfur-deprived, H2-producing Chlamydomonas reinhardtii cultures. *Plant Cell Physiol,* 2003, vol. 44, 146-155
- **KOSOUROV SN ; SEIBERT M.** Hydrogen photoproduction by nutrient-deprived Chlamydomonas reinhardtii cells immobilized within thin alginate films under aerobic and anaerobic conditions. *Biotechnol Bioeng,* 2009, vol. 102, 50-58
- **LAKATOS G ; DEAK Z ; VASS I ; RÉTFALVI T ; ROZGONYI S ; RÁKHELY G ; ÖRDÖG V ; KONDOROSI E ; MARÓTI G.** Bacterial symbionts enhance photo-fermentative hydrogen evolution of Chlamydomonas algae. *Green Chemistry,* 2014, vol. 16, 4716-4727
- **LELIAERT F ; VERBRUGGEN H ; ZECHMAN W.** Into the deep: New discoveries at the base of the green plant phylogeny. *Bioessays,* 2011, vol. 33, 683-692
- **MARIN B.** Nested in the Chlorellales or independent class? Phylogeny and classification of the Pedinophyceae (Viridiplantae) revealed by molecular phylogenetic analyses of complete nuclear and plastid-encoded rRNA operons. *Protist,* 2012, vol. 163, 778-805
- **MEHLER A.** Studies on the reactions of illuminated chloroplasts. II. Stimulation on inhibition of the reaction with molecular oxygen. *Arch Biochem Biophys,* 1951, vol. 34, 339-351
- **MELIS A ; ZHANG L ; FORESTIER M ; GHIRARDI ML ; SEIBERT M.** Sustained photobiological hydrogen gas production upon reversible inactivation of oxygen evolution in the green alga Chlamydomonas reinhardtii. *Plant Physiol,* 2000, vol. 122, 127-135
- **NAGY V ; VIDAL-MEIRELES A ; TENGÖLICS R ; RÁKHELY G ; GARAB G ; KOVACS L ; TÓTH SZ.** Ascorbate accumulation during sulphur deprivation and its effects on photosystem II activity and H2 production of the green alga Chlamydomonas reinhardtii. *Plant Cell Environ,* 2016, vol. 39, 1460-1472
- **ONCEL S ; KOSE A.** Comparison of tubular and panel type photobioreactors for biohydrogen production utilizing Chlamydomonas reinhardtii considering mixing time and light intensity. *Biores Technol,* 2014, vol. 151, 265-270
- **PINTO TS ; MALCATA FX ; ARRABAÇA JD ; SILVA JM ; SPREITZER RJ ; ESQUIVEL MG.** Rubisco mutants of Chlamydomonas reinhardtii enhance photosynthetic hydrogen production. *Appl Microbiol Biotechnol,* 2013, vol. 97, 5635-5643
- **PLANCKE C ; VIGEOLAS H ; HÖHNER R ; ROBERTY S ; EMONDS-ALT B ; LAROSA V ; WILLAMME R ; DUBY F ; ONGA DHALI D ; THONART P.** Lack of isocitrate lyase in Chlamydomonas leads to changes in carbon metabolism and in the response to oxidative stress under mixotrophic growth. *Plant J,* 2014, vol. 77, 404-417
- **PORRA RJ ; THOMPSON WA ; KRIEDEMAN PE.** Determination of accurate extinction coefficients and simultaneous equations for essaying chlorophylls-a and -b with four different solvents: verification of the concentration of chlorophyll standards by atomic absorption spectroscopy. *Biochim Biophys Acta,* 1989, vol. 975, 384-394
- **REEVES M ; GREENBAUM E.** Long-term endurance and selection studies in hydrogen and oxygen photoproduction by Chlamydomonas reinhardtii. *Enzyme Microb Technol,* 1985, vol. 7, 169-174
- **ROSENKRANS AM ; KRASNA AI.** Stimulation of hydrogen photoproduction in algae by removal of oxygen by reagents that combine reversibly with oxygen. *Biotechnol Bioeng,* 1984, vol. 26, 1334-1342
- **SCHANSKER G ; TÓTH SZ ; HOLZWARTH AR ; GARAB G.** Chlorophyll a fluorescence: beyond the limits of the QA model. *Photosynthesis Research,* 2014, vol. 120, 43-58
- **SCHÖTTLER MA ; TÓTH SZ.** Photosynthetic complex stoichiometry dynamics in higher plants: environmental acclimation and photosynthetic flux control. *Frontiers Plant Sci,* 2014, vol. 5, 188
- **SCOMA A ; DURANTE L ; BERTIN L ; FAVA F.** Acclimation to hypoxia in Chlamydomonas reinhardtii Can biophotolysis be the major trigger for long-term H2 production?. *New Phytol.,* 2014, vol. 204, 890-900
- **SCOMA A ; KRAWIETZ D ; FARALONI C ; GIANNELLI L ; HAPPE T ; TORZILLO G.** Sustained H2 production in a Chlamydomonas reinhardtii D1 protein mutant. *J Biotechnol,* 2012, vol. 157, 613-619
- **SCOMA A ; TÓTH SZ.** *On the pathways feeding the H2 production process in nutrient-replete, hypoxic conditions,* 2017
- **JURADO-OLLER et al.** Low oxygen levels contribute to improve photohydrogen production in mixotrophic non-stressed Chlamydomonas cultures. *Biotechnology for Biofuels,* 07 September 2015, vol. 8, 149
- *Biotechnol Biofuels,* vol. 10, 116
- **STEINBECK J ; NIKOLOVA D ; WEINGARTEN R ; JOHNSON X ; RICHAUD P ; PELTIER G ; HERMANN M ; MAGNESCHI L ; HIPPLER M.** Deletion of Proton Gradient Regulation 5 (PGR5) and PGR5-Like 1 (PGRL1) proteins promote sustainable light-driven hydrogen production in Chlamydomonas reinhardtii due to increased PSII activity under sulfur deprivation. *Front Plant Sci,* 2015, vol. 6, 892

- **SUNDBERG J ; CAMERON LJ ; SOUTHON PD ; KEPERT CJ ; MCKENZIE CJ.** Oxygen chemisorption/desorption in a reversible single-crystal-to-single-crystal transformation. *Chem Sci,* 2014, vol. 5, 4017-4025
- **SURZYCKI R ; COURNAC L ; PELTIER G ; RO-CHAIX J-D.** Potential for hydrogen production with inducible chloroplast gene expression in Chlamydomonas. *Proc Nat Acad Sci USA,* 2007, vol. 104, 17548-17553
- **SWANSON KD ; RATZLOFF MW ; MULDER DW ; ARTZ JH ; GHOSE S ; HOFFMAN A ; WHITE S ; ZADVORNYY OA ; BRODERICK JB ; BOTHNER B.** FeFe]-hydrogenase oxygen inactivation is initiated at the H Cluster 2Fe subcluster. *J Am Chem Soc,* 2015, vol. 137, 1809-1816
- **VIDAL-MEIRELES A ; NEUPERT J ; ZSIGMOND L ; ROSADO-SOUZA L ; KOVACS L ; NAGY V ; GALAMBOS A ; FERNIE AR ; BOCK R ; TÓTH SZ.** Regulation of ascorbate biosynthesis in green algae has evolved to enable rapid stress-induced response the VTC2 gene encoding GDP-L-galactose phosphorylase. *New Phytologist,* 2017
- **YAMORI W ; TAKAHASHI S ; MAKINO A ; PRICE D ; BADGER MR ; VON CAEMMERER S.** The roles of ATP synthase and the cytochrome b6/f complexes in limiting chloroplast electron transport and determining photosynthetic capacity. *Plant Physiol,* 2011, vol. 155, 956-962
- **ZHANG L. ; HAPPE T. ; MELIS A.** Biochemical and morphological characterization of sulfur-deprived and H2-producing Chlamydomonas reinhardtii (green alga). *Planta,* 2002, vol. 214, 552-561